# EUROPÄISCHE PATENTANMELDUNG

(11) **EP 4 755 340 A2**
(43) Veröffentlichungstag der Anmeldung: **10.06.2026**
(21) Anmeldenummer: 26165060.0
(22) Anmeldetag: 16.01.2012
(51) Int. Cl.: A61B 90/90

(54) **VERFAHREN ZUM DURCHFÜHREN EINER ABFRAGE EINES SPEZIFIKATIONSMERKMALS EINER MEDIZINTECHNISCHEN FUNKTIONSEINRICHTUNG, MEDIZINTECHNISCHE FUNKTIONSEINRICHTUNG, MEDIZINTECHNISCHE BEHANDLUNGSVORRICHTUNG SOWIE STEUEREINRICHTUNG**

(30) Priorität: 18.01.2011 DE 102011008856; 18.01.2011 US 201161433533 P
(62) Teilanmeldung aus: 22196182.4
(71) Anmelder: Fresenius Medical Care Deutschland GmbH, 61352 Bad Homburg (DE)
(72) Erfinder: Beden, Josef, verstorben (DE); Klewinghaus, Juergen, 61352 Bad Homburg (DE)
(74) Vertreter: Bobbert & Partner Patentanwälte PartmbB

(57) **Zusammenfassung**

Die vorliegende Erfindung betrifft ein Verfahren zum Durchführen einer Abfrage eines Spezifikationsmerkmals, welches in, an oder auf einer medizintechnischen Funktionseinrichtung angeordnet ist, mit dem Schritt: Abfragen des Spezifikationsmerkmals mittels einer mit der medizintechnischen Funktionseinrichtung funktionell gekoppelten oder zu koppelnden medizintechnischen Behandlungsvorrichtung oder einer hiermit in Signalübertragung verbundenen oder stehenden Einrichtung. Sie betrifft ferner eine medizintechnische Funktionseinrichtung, eine medizintechnische Behandlungsvorrichtung sowie eine Steuereinrichtung. Des Weiteren betrifft die vorliegende Erfindung ein digitales Speichermedium, ein Computer-Programm-Produkt sowie ein Computer-Programm.

## Beschreibung

Die vorliegende Erfindung betrifft ein Verfahren wie hierin offenbart zum Durchführen einer Abfrage eines Spezifikationsmerkmals, welches an einer medizintechnischen Funktionseinrichtung angeordnet ist. Sie betrifft ferner eine medizintechnische Funktionseinrichtung gemäß Anspruch 1, eine medizintechnische Behandlungsvorrichtung wie hierin offenbart sowie eine Steuereinrichtung wie hierin offenbart. Des Weiteren betrifft die vorliegende Erfindung ein digitales Speichermedium wie hierin offenbart, ein Computer-Programm-Produkt wie hierin offenbart sowie ein Computer-Programm wie hierin offenbart.

Medizintechnische Funktionseinrichtungen sind regelmäßig zu ihrem Einsatz in bestimmten Behandlungsverfahren geeignet. Diese Eignung kann durch ein an der Funktionseinrichtung angeordnetes Spezifikationsmerkmal kenntlich gemacht werden.

Eine Aufgabe der vorliegenden Erfindung ist es, ein Verfahren bereitzustellen, mittels welchem sich die Sicherheit erhöhen lässt, dass zum Durchführen einer mittels einer medizintechnische Behandlungsvorrichtung durchgeführten Behandlung eine hierzu tatsächlich geeignete medizintechnische Funktionseinrichtungen zum Einsatz kommt, nicht aber einer ungeeignete oder eine nicht ausreichend kompatible Funktionseinrichtung. Ferner sollen eine hierzu verwendbare medizintechnische Funktionseinrichtung, eine geeignete medizintechnische Behandlungsvorrichtung, eine geeignete Steuereinrichtung, ein geeignetes digitales Speichermedium, ein geeignetes Computer-Programm-Produkt und ein geeignetes Computer-Programm angegeben werden.

Die erfindungsgemäße Aufgabe wird durch ein Verfahren mit den Merkmalen wie hierin offenbart gelöst. Sie wird ferner durch eine medizintechnische Funktionseinrichtung mit den Merkmalen gemäß Anspruch 1, eine medizintechnische Behandlungsvorrichtung mit den Merkmalen wie hierin offenbart sowie eine Steuereinrichtung mit den Merkmalen wie hierin offenbart gelöst. Die erfindungsgemäße Aufgabe wird ferner gelöst durch ein digitales Speichermedium, ein Computer-Programm-Produkt sowie ein Computer-Programm wie hierin jeweils offenbart.

Alle mit dem erfindungsgemäßen Verfahren erzielbaren Vorteile lassen sich in bestimmten erfindungsgemäßen Ausführungsformen ungeschmälert auch mit der erfindungsgemäßen medizintechnischen Funktionseinrichtung und/oder mit der erfindungsgemäßen medizintechnischen Behandlungsvorrichtung und/oder mit der erfindungsgemäßen Steuereinrichtung erzielen. Dies gilt in einigen erfindungsgemäßen Ausführungsformen auch für das erfindungsgemäße digitale Speichermedium, das erfindungsgemäße Computer-Programm-Produkt sowie das erfindungsgemäße Computer-Programm.

Das erfindungsgemäße Verfahren ist geeignet und vorgesehen zum Durchführen einer Abfrage eines Spezifikationsmerkmals, welches in, an oder auf einer medizintechnischen Funktionseinrichtung angeordnet ist. Es umfasst das Abfragen des Spezifikationsmerkmals mittels einer mit der medizintechnischen Funktionseinrichtung funktionell gekoppelten oder zu koppelnden medizintechnischen Behandlungsvorrichtung oder einer hiermit in Signalübertragung stehenden oder in Signalverbindung verbundenen Einrichtung.

Die erfindungsgemäße medizintechnische Funktionseinrichtung (im Folgenden auch kurz: Funktionseinrichtung) weist wenigstens ein Spezifikationsmerkmal auf, welches wenigstens eine, insbesondere spezifische oder konkrete, medizintechnische Funktion oder Eignung der medizintechnischen Funktionseinrichtung kennzeichnet. Dabei ist das Spezifikationsmerkmal in, an oder auf der medizintechnischen Funktionseinrichtung angebracht, vorgesehen, geeignet und/oder konfiguriert, um mittels einer mit der medizintechnischen Funktionseinrichtung funktionell gekoppelten oder zu koppelnden medizintechnischen Behandlungsvorrichtung oder einer hiermit in Signalübertragung verbundenen oder stehenden Einrichtung abgefragt zu werden.

Die erfindungsgemäße medizintechnische Behandlungsvorrichtung (im Folgenden auch kurz: Behandlungsvorrichtung) weist wenigstens eine Erfassungseinrichtung auf und/oder ist hiermit in Signalübertragung verbunden oder steht mit dieser in einer Signalübertragungsbeziehung. Die Erfassungseinrichtung ist dazu geeignet, vorgesehen und/oder konfiguriert, das wenigstens eine Spezifikationsmerkmal zu erfassen, welches wenigstens eine Funktion der medizintechnischen Funktionseinrichtung kennzeichnet.

Die erfindungsgemäße Steuereinrichtung ist geeignet und vorgesehen und/oder ausgelegt und/oder konfiguriert zur Durchführung des erfindungsgemäßen Verfahrens.

Ein erfindungsgemäßes digitales Speichermedium, insbesondere in Form einer Diskette, CD oder DVD, mit elektrisch auslesbaren Steuersignalen kann derart mit einem programmierbaren Computersystem zusammenwirken, dass die maschinellen Schritte eines erfindungsgemäßen Verfahrens veranlasst werden.

Dabei können alle, einige oder manche der maschinell durchgeführten Schritte des erfindungsgemäßen Verfahrens veranlasst werden.

Ein erfindungsgemäßes Computer-Programm-Produkt weist einen auf einem maschinenlesbaren Träger gespeicherten Programmcode zur Veranlassung der maschinellen Schritte des erfindungsgemäßen Verfahrens, wenn das Computer-Programm-Produkt auf einem Rechner abläuft, auf.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein erfindungsgemäßes Computer-Programm weist einen Programmcode auf zur Veranlassung der maschinellen Schritte eines erfindungsgemäßen Verfahrens, wenn das Computer-Programm auf einem Computer abläuft.

Auch für das erfindungsgemäße Computer-Programm-Produkt und das erfindungsgemäße Computer-Programm gilt, dass alle, einige oder manche der maschinell durchgeführten Schritte des erfindungsgemäßen Verfahrens veranlasst werden.

Vorteilhafte Weiterbildungen der vorliegenden Erfindung sind jeweils Gegenstand der Unteransprüche.

Erfindungsgemäße Ausführungsformen können manche, einige oder alle der folgenden Merkmale in beliebiger Kombination aufweisen.

Ein "Abfragen" bezeichnet in bestimmten Ausführungsformen der vorliegenden Erfindung das Feststellen und/oder Gewinnen und/oder Treffen einer Aussage und/oder einer Information oder einer Angabe oder dergleichen über das Spezifikationsmerkmal.

Solche Aussagen, Informationen oder Angaben betreffen in manchen Ausführungsformen das Zutreffen, Vorliegen oder Vorhandensein, oder aber eben das Nicht-Zutreffen, Nicht-Vorliegen oder Nicht-Vorhandensein des Spezifikationsmerkmals selbst, oder eines bestimmten Zustands, eines bestimmten Merkmals oder einer bestimmten Ausgestaltung oder dergleichen des Spezifikationsmerkmals.

In manchen Ausführungsformen betreffen solche Aussagen, Informationen oder Angaben eine bestimmte, vorbestimmte, definierte, gewünschte, und/oder erforderliche Qualität, Quantität, Ausprägung und/oder Eigenschaft und dergleichen des Spezifikationsmerkmals.

In bestimmten Ausführungsformen der vorliegenden Erfindung kennzeichnet das Spezifikationsmerkmal eine Funktion oder Eignung zu einer Verwendung (oder auch mehrere Funktionen oder Eignungen zu verschiedenen Verwendungen) der Funktionseinrichtung. In solchen Ausführungsformen betrifft das Abfragen des Spezifikationsmerkmals eine Abfrage der wenigstens einen Funktion oder Eignung der Funktionseinrichtung.

In bestimmten Ausführungsformen ist das Spezifikationsmerkmal geeignet, vorgesehen und/oder konfiguriert, um von der Behandlungsvorrichtung bzw. einer mit der Behandlungsvorrichtung in Signalübertragung stehenden oder verbundenen Einrichtung hinsichtlich seiner Informationen oder Angaben über die spezifizierende Funktion (oder Funktionen) der Funktionseinrichtung detektiert, erfasst oder abgefragt zu werden. Die Behandlungsvorrichtung kann eigens vorbereitet oder konfiguriert sein, um das Spezifikationsmerkmal in seiner konkreten Ausgestaltung detektieren, erfassen oder abfragen zu können.

Die Begriffe Funktion und Eignung sind in bestimmten Ausführungsformen als gleichbedeutend und/oder gegeneinander austauschbar zu verstehen.

Eine Funktion oder Eignung einer medizintechnischen Funktionseinrichtung bezeichnet in bestimmten Ausführungsformen der vorliegenden Erfindung die Fähigkeit der medizintechnischen Funktionseinrichtung, einen bestimmten Vorgang, Prozess, Ablauf und dergleichen, insbesondere im Rahmen einer medizinischen Behandlung, ausführen zu können oder einen bestimmten Nutzen bieten zu können.

In bestimmten Ausführungsformen ist die medizintechnische Funktionseinrichtung zur Ausführung eines bestimmten Vorgangs, Prozesses, Ablaufs usw. konzipiert und/oder vorgesehen und/oder ausgestaltet.

Der Begriff "funktionelle Kopplung" oder "funktionell gekoppelt", wie er hierin in bestimmten Ausführungsformen der vorliegenden Erfindung verwendet wird, bezeichnet die Herstellung oder Etablierung einer Wirkverbindung zwischen Elementen. Beispiele für geeignete Wirkverbindungen schließen, ohne auf die im Folgenden genannten beschränkt zu sein, elektrische, elektronische, mechanische, pneumatische, magnetische, induktive, optische, strömungsrelevante, und dergleichen, Verbindungen ein.

Eine funktionelle Kopplung der Funktionseinrichtung mit der Behandlungsvorrichtung bezeichnet in bestimmten Ausführungsformen eine Anordnung der Funktionseinrichtung und der Behandlungsvorrichtung, ohne welche die Behandlungsvorrichtung bestimmte oder wesentliche Behandlungsoptionen nicht ausführen kann.

Eine mit der Behandlungsvorrichtung in Signalübertragung verbundene Einrichtung steht in bestimmten Ausführungsformen der vorliegenden Erfindung in Signalübertragungskommunikation (unter körperlichem Kontakt oder kontaktlos) mit der Behandlungsvorrichtung.

Die hierzu verwendeten Signale können Funksignale, Infrarot-Signale, Signale via Bluetooth, Radiowellen, elektrische Signale und dergleichen sein. Diese Beispiele sind keinesfalls einschränkend zu verstehen.

Ein Signal bezeichnet in bestimmten Ausführungsformen übermittelte Informationen hinsichtlich des abgefragten Spezifikationsmerkmals oder impliziert oder erlaubt Rückschlüsse hierauf.

Das Signal kann dabei jede geeignete Art von Signal wie ein Analogsignal, ein Digitalsignal und dergleichen mehr, Kombinationen, Mischungen usw. sein.

Das Abfragen des Spezifikationsmerkmals mittels der Behandlungsvorrichtung oder einer hiermit in Signalübertragung verbundenen Einrichtung erfolgt in bestimmten Ausführungsformen mittels einer Steuereinrichtung, einer CPU oder dergleichen der Behandlungsvorrichtung.

In bestimmten erfindungsgemäßen Ausführungsformen ist das Spezifikationsmerkmal als Markierung oder als Etikett ausgestaltet und/oder weist wenigstens eine Markierung oder ein Etikett auf.

Beispiele für geeignete Markierungen schließen - rein exemplarisch und nicht einschränkend - Dielektrika, metallische Objekte, Leiter, Permanentmagneten, Schwingkreise, Aufsätze bzw. Vorsprünge oder Einrückungen oder Kerben, Blenden/Optiken, reflektierende und/oder lichtabsorbierende und/oder Licht streuende Objekte, farbige Objekte, Barcodes/Muster, polarisierende Objekte, dämpfende Objekte oder Schwingungen verstärkende Objekte ein.

In bestimmten Ausführungsformen ist das Spezifikationsmerkmal ausgestaltet als Barcode, Dot-Matrix-Code, RFID-Chip oder Farbmarkierung.

Beliebige Kombination der hierin genannten und/oder weiterer geeigneter Markierungen sind ebenfalls von der vorliegenden Erfindung umfasst.

Das Spezifikationsmerkmal kann lösbar oder auch unlösbar, insbesondere vorzugsweise unlösbar während des Kopplungsvorgang der Funktionseinrichtung mit der Behandlungsvorrichtung und/oder während des Abfragevorgangs des Spezifikationsmerkmals, mit der Funktionseinrichtung verbunden oder in, an oder auf der Funktionseinrichtung befestigt sein.

In bestimmten Ausführungsformen ist das Spezifikationsmerkmal vorgesehen zur Befestigung an der Funktionseinrichtung mittels Aufkleben, Aufdrucken, Beschichten, Einfärben, Durchfärben, Verschnappen, Verrasten und dergleichen, oder befestigt.

Das Spezifikationsmerkmal kann auf einem Trägermaterial angeordnet sein oder nicht. Das Spezifikationsmerkmal kann direkt auf oder an der Funktionseinrichtung angeordnet oder angebracht bzw. befestigt sein. Das Spezifikationsmerkmal kann Bestandteil der Funktionseinrichtung sein.

In Ausführungsformen, in denen das Spezifikationsmerkmal unter Verwenden eines Trägermaterials in, auf oder an der Funktionseinrichtung befestigt ist oder wird, kann das Trägermaterial durchsichtig oder undurchsichtig gewählt sein. Es kann hiervon abweichend einen - gegenüber der Funktionseinrichtung bzw. Elementen derselben - farblich abgegrenzten, Hintergrund aufweisen.

In bestimmten Ausführungsformen der vorliegenden Erfindung ist das Spezifikationsmerkmal vorgesehen zur Kennzeichnung eines extrakorporalen Blutschlauchsatzes oder einer bestimmten Ausgestaltung oder Eignung des extrakorporalen Blutschlauchsatzes. Beispiele für solche Ausgestaltungen oder Eignungen des extrakorporalen Blutschlauchsatzes umfassen eine Ausgestaltung oder Eignung für bestimmte medizinische Zwecke, wie beispielsweise eine Blutbehandlung z. B. bei einer Hämodialyse, Hämofiltration oder Hämodiafiltration, mit oder ohne Zugabe von Substituatflüssigkeit, und ggf. Zugabe wenigstens eines Antikoagulans und/oder weiterer Blutadditive und dergleichen.

Das erfindungsgemäße Verfahren umfasst in manchen Ausführungsformen das Auswählen einer medizinischen Behandlungsoption an der Behandlungsvorrichtung.

Eine Behandlungsoption bezeichnet in bestimmten Ausführungsformen der vorliegenden Erfindung eine Behandlungsmöglichkeit (von oftmals mehreren, verschiedenen Behandlungsmöglichkeiten), eine Behandlungsalternative, einen bestimmten Behandlungsschritt, eine Abfolge von Schritten oder Prozessen, jeweils im Rahmen eines Behandlungsverfahrens, beispielsweise eines Blutbehandlungsverfahrens.

Ein "Auswählen einer Behandlungsoption" bezeichnet in manchen Ausführungsformen der vorliegenden Erfindung das Bestimmen oder Festlegen einer bestimmten Behandlungsoption aus einer Mehr- oder Vielzahl verschiedener, mittels der Behandlungsvorrichtung durchführbaren Behandlungsoptionen.

Die Auswahl kann manuell durch Eingabe der gewünschten und/oder erforderlichen Behandlungsoption in die Behandlungsvorrichtung bzw. eine zu diesem Zweck geeignete und vorgesehene (Eingabe-) Einrichtung, z. B. eine Tastatur, eine Maus, einen Druckknopf und dergleichen, z. B. durch eine entsprechend autorisierte und/oder qualifizierte Person, erfolgen.

Die Auswahl kann automatisch oder automatisiert, aufgrund oder im Anschluss an eine Vorgeschichte des bisherigen Behandlungsverfahrens, erfolgen.

Die Auswahl kann vor, während oder nach dem Abfragen des Spezifikationsmerkmals erfolgen.

Die zur Auswahl stehenden Behandlungsoptionen können in der Behandlungsvorrichtung, beispielsweise einer Speichereinrichtung der Behandlungsvorrichtung, hinterlegt sein oder werden.

Das erfindungsgemäße Verfahren umfasst in bestimmten Ausführungsformen das In-Kontakt-Bringen und/oder Verbinden der Funktionseinrichtung mit der Behandlungsvorrichtung oder einer Erfassungseinrichtung der Behandlungsvorrichtung.

Ein "In-Kontakt-Bringen" und/oder "Verbinden" bezeichnet in bestimmten Ausführungsformen der vorliegenden Erfindung ein räumliches Anordnen oder Nahebringen der Funktionseinrichtung und der Behandlungsvorrichtung zu- bzw. miteinander. Beides dient dem Abfragen des Spezifikationsmerkmals oder ermöglicht ein solches. Im Gegensatz zum Begriff des "(funktionellen)" Koppelns ist hier das In-Kontakt-Bringen oder Verbinden der Funktionseinrichtung und der Behandlungsvorrichtung als bloßes Einander-Nahe-Bringen, z. B. in Form eines Dranhaltens, oder auch in Gestalt eines mechanischen Verbindens, zu verstehen. Das Etablieren einer abschließenden Wirkverbindung ist hierzu nicht erforderlich, allerdings auch nicht ausgeschlossen.

Die räumliche Anordnung der Funktionseinrichtung und der Behandlungsvorrichtung erlaubt bzw. dient in bestimmten Ausführungsformen einer Abfrage des Spezifikationsmerkmals durch die Behandlungsvorrichtung oder eine entsprechende Einrichtung aufgrund der geschaffenen räumlichen Nähe.

In bestimmten Ausführungsformen umfasst das erfindungsgemäße Verfahren das Erfassen des Spezifikationsmerkmals mittels der Behandlungsvorrichtung oder einer hiermit in Signalübertragung verbundenen Erfassungseinrichtung.

Eine Erfassungseinrichtung bezeichnet in bestimmten Ausführungsformen der vorliegenden Erfindung eine Einrichtung, welche geeignet und vorgesehen und/oder ausgestaltet ist zum Erfassen und/oder Messen des einen Spezifikationsmerkmals (oder mehrerer Spezifikationsmerkmale) der medizintechnischen Funktionseinrichtung.

Die Erfassungseinrichtung ist an der Behandlungsvorrichtung vorgesehen oder in diese eingebaut. In bestimmten Ausführungsformen liegt die Erfassungseinrichtung als separates Bauteil vor; in anderen Ausführungsformen ist die Erfassungseinrichtung oder ein Abschnitt desselben Teils der Behandlungsvorrichtung, z. B. Teil des Gehäuses der Behandlungsvorrichtung.

In bestimmten Ausführungsformen ist die Erfassungseinrichtung dazu ausgelegt und/oder vorgesehen, das zum Erfassen und/oder Messen des Spezifikationsmerkmals gewünschte und/oder erforderliche Messsignal selbst zu erzeugen oder anzuregen.

In manchen Ausführungsformen ist die Erfassungseinrichtung zu diesem Zweck mit einer geeigneten, dazu vorgesehenen und/oder konfigurierten Signalerzeugungseinrichtung ausgestaltet oder (physisch) verbunden oder steht in Wirkverbindung mit einer solchen.

Geeignete Signalerzeugungseinrichtungen umfassen Permanentmagneten, Oszillatoren, Kondensatoren, Spannungsquellen, Wärmequellen, Teilchenstrahler, Laser, Lichtquellen wie Weißlicht-LED oder RGB-LED (für: rotes, grünes und blaues Licht emittierende LEDs, LED für: light emitting diode), IRED (für infra red emitting diode), weitere oder andere Lichtquellen, Piezoelemente oder dergleichen.

Geeignete, zu erfassende Messsignale zum Erfassen und/oder Messen des Spezifikationsmerkmals schließen optische Messsignale, wie Intensität, Frequenz (beispielsweise Radiofrequenz), Wellenlänge, Licht (wie Reflexionslicht), elektrische Messsignale, wie Spannung, Strom, Widerstand, Kapazität, Induktivität, elektrische Felder, magnetische Felder, mechanische Messsignale (wie Kraft, Druck, Weg), magnetische Messsignale (wie magnetische Feldstärke, Magnetfeldmessung, Magnetisierung), akustische Messsignale (wie Schalldruck, Schallgeschwindigkeit) und/oder chemische Messsignale und dergleichen ein.

Geeignete Erfassungseinrichtungen schließen solche ein, welche optische oder opto-elektronische Effekte, Kapazitativeffekte, Induktion oder Absorption/Dämpfung, Lichtsignale (Farbsensoren oder Weißlichtsensoren), Reflektion/Transmission, thermische Effekte, chemische Effekte und dergleichen mehr messen oder erfassen.

In einigen Ausführungsformen erfasst die Erfassungseinrichtung einen von dieser selbst an der Funktionseinrichtung oder dem Spezifikationsmerkmal zum Erfassen des Spezifikationsmerkmals initiierten Effekt; dieser ist beispielsweise ein Halleffekt, eine Magnetisierung, ein Thermowiderstandseffekt/thermoelektrischen Effekt, ein piezoelektrischen/-resistiven Effekt, ein Photoeffekt, eine Spannungs-/Stromänderung, ein Induktionseffekt oder dergleichen.

In bestimmten Ausführungsführungsformen der vorliegenden Erfindung ist die Erfassungseinrichtung ausgestaltet als Hallsensor, (Reed-) Relais, Volt-/Amperemeter, Drucksensor, elektrischer Schalter, Temperaturfühler, als Fotodiode oder Fotowiderstand.

In bestimmten Ausführungsformen der vorliegenden Erfindung ist die Erfassungseinrichtung ausgestaltet als Barcode-Lesegerät, Scanner, Farbsensor, wie als LED-Sensor, Chip-Lesegerät oder dergleichen, oder als eine Kombination derselben, oder sie weist solche auf.

In bestimmten weiteren Ausführungsformen umfasst das erfindungsgemäße Verfahren das Erkennen und/oder Zuordnen einer mit dem Spezifikationsmerkmal verbundenen Funktion.

Ein "Erkennen" oder "Zuordnen" bedeutet in bestimmten Ausführungsformen der vorliegenden Erfindung ein Wahrnehmen, Auswerten oder Beurteilen und dergleichen des von der Erfassungseinrichtung erfassten Spezifikationsmerkmals; das erkannte Spezifikationsmerkmal wird einer durch das Spezifikationsmerkmal spezifizierte oder angegebene Funktion der Funktionseinrichtung zugeordnet.

In bestimmten Ausführungsformen der vorliegenden Erfindung sind Spezifikationsmerkmale - bevorzugt und/oder sinnvollerweise mit den dazugehörigen, von den Spezifikationsmerkmalen spezifizierten Funktionen der Funktionseinrichtung(en) - in der Behandlungsvorrichtung oder einer hiermit in Datenübertragung stehende Einrichtung, z. B. einer Datenbank oder einer Speichereinrichtung der Behandlungsvorrichtung oder der Einrichtung, hinterlegt.

Das Erkennen und/oder Zuordnen des Spezifikationsmerkmals beinhaltet in bestimmten Ausführungsformen, das Vergleichen des aktuell erfassten Spezifikationsmerkmals mit den in der Behandlungsvorrichtung hinterlegten Spezifikationsmerkmalen.

In bestimmten Ausführungsformen umfasst das erfindungsgemäße Verfahren das Vergleichen der, z. B. an der medizintechnischen Behandlungsvorrichtung für den konkreten Behandlungsfall z. B. mittels Tastatur, ausgewählten Behandlungsoption mit der anhand des Spezifikationsmerkmals der Funktionseinrichtung von der Behandlungsvorrichtung erkannten Funktion oder Eignung.

Dieses Vergleichen umfasst in bestimmten Ausführungsformen ein Überprüfen, ob die mit dem Spezifikationsmerkmal der Funktionseinrichtung gekennzeichnete Funktion der Funktionseinrichtung mit der ausgewählten Behandlungsoption übereinstimmt, und/oder ob sie mit dieser zumindest kompatibel ist.

In bestimmten Ausführungsformen umfasst das Vergleichen das Treffen einer Aussage - und vorzugsweise zusätzlich deren Mitteilen an den Zuständigen bzw. den Benutzer - über das Zueinanderpassen der anhand des Spezifikationsmerkmals der Funktionseinrichtung erfassten Funktion der Funktionseinrichtung und der Behandlungsoption derart, dass eine Entscheidung über den Einsatz der Funktionseinrichtung im Rahmen der Behandlungsoption bzw. eines Behandlungsverfahrens getroffen werden kann.

In anderen Ausführungsformen umfasst das Vergleichen ein Verhindern, dass die ausgewählte Behandlungsoption mit der vorliegenden Funktionseinrichtung ausführbar ist. Um ein solches Ausführen mit einer als "unpassend" erkannten Funktionseinrichtung zu verhindern kann eine Sicherungsfunktion vorgesehen sein. Diese kann in einer Stromabschaltung oder ähnlichem bestehen.

In manchen Ausführungsformen umfasst das Vergleichen das Beurteilen einer Verträglichkeit und/oder Zulässigkeit der Verwendung einer bestimmten Funktionseinrichtung im Rahmen einer bestimmten Behandlungsoption bzw. eines bestimmten Behandlungsverfahrens. In bestimmten Ausführungsformen beinhaltet das Vergleichen das Feststellen einer Tauglichkeit oder - bevorzugt unkritischen - Verwendbarkeit der Funktionseinrichtung. Dies kann beispielsweise durch Vergleich mit gespeicherten Daten, z. B. in Tabellenform hinterlegten Daten, erfolgen.

Das Ergebnis eines Vergleichs kann ein "positiv" oder "negativ", ein "zutreffend" oder "nicht-zutreffend", ein "korrekt" oder "unkorrekt" oder dergleichen sein.

Im Falle eines positiven Ergebnisses des Vergleichs, d. h. im Allgemeinen nach Feststellung einer Übereinstimmung oder Kompatibilität zwischen erkanntem Spezifikationsmerkmal (und damit der Funktion der Funktionseinrichtung) und der ausgewählten Behandlungsoption, ist in bestimmten Ausführungsformen vorgesehen, eine anstehende Verwendung der Funktionseinrichtung in oder an oder in Verbindung mit der Behandlungsvorrichtung vorzubereiten oder zu veranlassen, z. B. mittels automatisierter Sicherheitsabfragen.

In bestimmten Ausführungsformen umfasst das Verfahren das funktionelle Koppeln der Funktionseinrichtung mit der Behandlungsvorrichtung, wenn die Behandlungsoption und die erkannte Funktion miteinander übereinstimmen und/oder wenn sie zumindest kompatibel sind.

Ein funktionelles Koppeln kann das Verbinden und/oder Zueinanderführen und dergleichen der Funktionseinrichtung und der Behandlungsvorrichtung unter Aufnahme einer Wirkverbindung, wie einer mechanischen Wirkverbindung, einer Verbindung zu Signalübertragungszwecken, einer elektrischen Verbindung und dergleichen umfassen. In bestimmten Ausführungsformen dient das funktionelle Koppeln dem Vorbereiten eines Behandlungsverfahrens mittels der Behandlungsvorrichtung unter Einsatz der Funktionseinrichtung.

In weiteren Ausführungsformen umfasst das erfindungsgemäße Verfahren das Starten definierter Abfragen bezüglich der korrekten funktionellen Ankopplung und/oder der zwischenzeitlich erzielten Einsetzbarkeit der Funktionseinrichtung für das geplante Behandlungsverfahren.

Eine "definierte Abfrage" bezeichnet in manchen Ausführungsformen der vorliegenden Erfindung ein Abfragen oder Überwachen bestimmter, mit der Verwendung der Funktionseinrichtung oder der Behandlungsvorrichtung verbundener Tätigkeiten.

In bestimmten Ausführungsformen sind die definierten Abfragen Sicherheitsabfragen, welche dazu vorgesehen sind, die Sicherheit des Systems - und damit vorzugsweise auch die Patientensicherheit - zu erhöhen.

Abfragen hinsichtlich der funktionellen Ankopplung und/oder der Einsetzbarkeit der Funktionseinrichtung an, auf oder mit der Behandlungsvorrichtung schließen Abfragen über einen korrekten Sitz der Funktionseinrichtung in der Behandlungsvorrichtung, die Korrektheit einer Anordnung Komponenten zueinander, eine korrekte Signalankopplung und/oder elektrische Verbindung zwischen zwei oder mehreren Komponenten, einen korrekten Verschluss- oder Öffnungszustand "geschlossen" oder "offen" (wie beispielsweise bei Schläuchen oder Klemmen oder Ventilen) von zu verwendenden Komponenten, insbesondere vor Beginn der Behandlung, und dergleichen mehr ein.

Fällt der o. g. Vergleich negativ aus, so umfasst das erfindungsgemäße Verfahren in bestimmten Ausführungsformen das Ausgeben eines Alarms und/oder einer Anzeige über ein Abweichen zwischen ausgewählter Behandlungsoption und erkannter Funktion. Dieses Ergebnis kann dann vorliegen, wenn die ausgewählte Behandlungsoption und die erkannte Funktion nicht übereinstimmen und/oder nicht miteinander kompatibel sind.

Geeignete Alarm- und/oder Anzeigeeinrichtungen zu diesem Zweck oder auch zu anderen, insbesondere mit dem Behandlungsverfahren verbundenen, Zwecken können entsprechend vorgesehen sein. Beispiele schließen eine (oder mehrere) akustische und/oder visuelle Alarme erzeugende Einrichtungen und/oder beispielsweise den Alarm oder einen Fehler bzw. ein Problem anzeigende Einrichtungen bzw. Anzeigen, wie Bildschirme, und dergleichen ein.

Die erfindungsgemäße Funktionseinrichtung ist vorgesehen zu ihrer funktionellen Kopplung mit einer Behandlungsvorrichtung. Sie ist geeignet und vorgesehen und/oder ausgestaltet zur Verwendung zur Durchführung wenigstens einer bestimmten Funktion, insbesondere im Rahmen eines medizinischen Behandlungsverfahrens und/oder einer medizinischen Behandlungsoption.

In bestimmten Ausführungsformen ist die Funktionseinrichtung ausgelegt zu ihrem Einsatz in oder während eines extrakorporalen Blutbehandlungsverfahrens.

Die erfindungsgemäße Funktionseinrichtung weist in bestimmten Ausführungsformen wenigstens einen extrakorporalen Blutschlauchsatz auf oder besteht hieraus.

In manchen erfindungsgemäßen Ausführungsformen weist die erfindungsgemäße Funktionseinrichtung einen in einem Tray oder Organizer angeordneten, extrakorporalen Blutschlauchsatz auf oder besteht hieraus.

In weiteren Ausführungsformen ist die medizintechnische Funktionseinrichtung als Kassette, z. B. als Disposable-Kassette, ausgestaltet.

Ein "Organizer" bezeichnet in bestimmten Ausführungsformen der vorliegenden Erfindung eine Einrichtung, welche vorgesehen ist zur Aufnahme und/oder kompakteren Handhabung der medizintechnischen Funktionseinrichtung, beispielsweise eines extrakorporalen Blutschlauchsatzes. Extrakorporaler Blutschlauchsatz und Organizer können in gemeinsamer Anordnung eine Kassette bilden oder so bezeichnet werden. Beispiele für geeignete Organizer können den

Ausführungsformen der deutschen Patentanmeldung DE 10 2008 026 915.8 mit dem Titel "Faltbare Organizer für Blutschlauchelemente" der vorliegenden Anmelderin entnommen werden, auf deren diesbezügliche Offenbarung hiermit vollinhaltlich Bezug genommen wird.

Ein extrakorporaler Blutschlauchsatz, wie er hierin verwendet wird, kann beispielsweise ein in einem Blutbehandlungsverfahren, wie einer Hämodialyse, einer Hämofiltration, einer Hämodiafiltration oder dergleichen, eingesetzter extrakorporaler Blutschlauchsatz sein. Er kann alle die zu einem solchen (oder einem anderen (Blut-) Behandlungszweck gewünschten und/oder erforderlichen Komponenten oder Elemente oder Einrichtungen aufweisen oder mit entsprechenden Komponenten oder Elementen oder Einrichtungen verbunden sein. Nicht-einschränkende und rein exemplarische Beispiele schließen Leitungen, Kammern, Schläuche, Klemmen, Sensoren wie Drucksensoren oder Luftblasendetektoren, Zugabeeinrichtungen, Ventile, Drosseleinrichtungen, den Durchfluss von Fluiden, beispielsweise Blut und/oder Dialysat und/oder Substituat, regulierende Einrichtungen, und dergleichen ein.

In bestimmten Ausführungsformen weist die Funktionseinrichtung wenigstens eine Zugabestelle für Calcium und wenigstens eine Zugabestelle für Citrat auf. Die Calcium-und die Citrat-Zugabestelle sind vorgesehen zur Zugabe von Citrat- und Calciumlösungen oder anderen, nach gleichem oder ähnlichem Wirkprinzip wirkende Antikoagulantien, in die Funktionseinrichtung oder in den extrakorporalen Blutschlauchsatz.

In bestimmten weiteren Ausführungsformen weist die Funktionseinrichtung ferner wenigstens eine Heparin-Zugabestelle auf. Die Heparin-Zugabestelle ist vorgesehen zur Zugabe von Heparin oder anderen, nach gleichem oder ähnlichem Wirkprinzip wirkende Antikoagulantien, in die Funktionseinrichtung oder in den extrakorporalen Blutschlauchsatz.

Neben Heparin und/oder Calcium und Citrat als Antikoagulantien kann die Zugabe weiterer Antikoagulantien oder das zu behandelnde Blut beeinflussende Substanzen im Rahmen des Behandlungsverfahrens vorgesehen sein. Geeignete Einrichtungen können jeweils vorgesehen sein.

Die Zugabe von Heparin oder Calcium und Citrat kann mittels Pumpen erfolgen. Entsprechende, hierzu geeignete und/oder vorgesehene Pumpen sind in bestimmten erfindungsgemäßen Ausführungsformen vorgesehen.

Die erfindungsgemäße medizintechnische Behandlungsvorrichtung ist geeignet und vorgesehen und/oder ausgestaltet zur Durchführung eines oder mehrerer Schritte eines medizinischen Behandlungsverfahrens und/oder einer oder mehrerer Behandlungsoptionen im Rahmen eines medizinischen Behandlungsverfahrens.

Die medizintechnische Behandlungsvorrichtung mit wenigstens einer medizintechnischen Funktionseinrichtung funktionell koppelbar oder gekoppelt bzw. hierzu jeweils vorgesehen.

Die Behandlungsvorrichtung weist in bestimmten Ausführungsformen wenigstens eine Erkennungseinrichtung auf, welche dazu geeignet, vorgesehen und/oder konfiguriert ist, anhand des von der Erfassungseinrichtung erfassten Spezifikationsmerkmals eine mit der Funktionseinrichtung verbundene und/oder dieser zugeordnete Funktion zu erkennen und/oder zuzuordnen.

Hierzu können, wie vorstehend ausgeführt, in bestimmten Ausführungsformen der vorliegenden Erfindung Informationen über die zu einem bestimmten Spezifikationsmerkmal gehörende Funktion oder Eignung der Funktionseinrichtung in der Behandlungsvorrichtung bzw. einer Speichereinrichtung derselben hinterlegt sein oder hinterlegt werden.

In bestimmten Ausführungsformen weist die Behandlungsvorrichtung ferner wenigstens eine Vergleichseinrichtung auf. Die Vergleichseinrichtung ist dazu geeignet, vorgesehen und/oder konfiguriert, eine an der medizintechnischen Behandlungsvorrichtung ausgewählte Behandlungsoption mit der anhand des Spezifikationsmerkmals erkannten Funktion oder Eignung der medizintechnischen Funktionseinrichtung zu vergleichen.

In manchen Ausführungsformen weist die Behandlungsvorrichtung ferner wenigstens eine Abfrageeinrichtung auf, welche dazu geeignet, vorgesehen und/oder konfiguriert ist, definierte und/oder vorbestimmte Abfragen bezüglich der korrekten funktionellen Ankopplung und/oder der Einsetzbarkeit der Funktionseinrichtung im geplanten Behandlungsverfahren und/oder im Rahmen der ausgewählten Behandlungsoption durchzuführen oder deren Durchführung zu veranlassen. Diese Abfragen können insbesondere Sicherheitsabfragen, vor allem herstellerseitig oder gesetzlich bzw. durch eine Norm vorgeschriebene oder empfohlene, sein.

In weiteren bestimmten Ausführungsformen weist die Behandlungsvorrichtung ferner wenigstens eine Alarmeinrichtung und/oder eine Anzeigeeinrichtung auf, welche vorgesehen sind zum Ausgeben eines Alarms oder einer Anzeige bei Nicht-Kompatibilität des Spezifikationsmerkmals bzw. der Funktion der Funktionseinrichtung oder der Funktionseinrichtung selbst und der Behandlungsvorrichtung bzw. der ausgewählten Behandlungsoption. Dies kann insbesondere in Fällen sinnvoll und/oder gewünscht und/oder erforderlich sein, in denen eine nicht zu der ausgewählten Behandlungsoption passende Funktionseinrichtung gewählt und irrtümlicherweise mit der Behandlungsvorrichtung konnektiert werden sollte. Ein - insbesondere bevorzugt rechtzeitig vor einem funktionellen Koppeln der Funktionseinrichtung an die Behandlungsvorrichtung oder gar dem Starten des Behandlungsverfahrens - ausgegebener Alarm kann in bestimmten Ausführungsformen vorteilhaft dazu beitragen, einen nicht vorgesehenen Einsatz der Funktionseinrichtung zu vermeiden und damit vorteilhaft die Sicherheit für den Patienten zu erhöhen.

Weitere Alarme und/oder Anzeigen andere Situationen im Rahmen eines Behandlungsverfahrens betreffend können ebenso wie entsprechende Einrichtungen ebenfalls vorgesehen sein.

Die erfindungsgemäße Steuereinrichtung weist wenigstens eine Einrichtung auf oder ist funktionell gekoppelt und/oder in Signalübertragung verbunden mit einer Einrichtung, die ausgewählt ist aus einer Gruppe, aufweisend oder bestehend aus einer Erfassungseinrichtung, einer Erkennungseinrichtung, einer Vergleichseinrichtung, einer Abfrageeinrichtung, einer Alarmeinrichtung und einer Anzeigeeinrichtung.

In manchen Aspekten betrifft die Erfindung ein Verfahren zum Durchführen einer Abfrage eines Spezifikationsmerkmals (400), welches in, an oder auf einer medizintechnischen Funktionseinrichtung (300) angeordnet ist, mit dem Schritt:
- Abfragen des Spezifikationsmerkmals (400) mittels einer mit der medizintechnischen Funktionseinrichtung (300) funktionell gekoppelten oder zu koppelnden medizintechnischen Behandlungsvorrichtung (100) oder einer hiermit in Signalübertragung verbundenen oder stehenden Einrichtung.

In einigen Aspekten der Erfindung erfolgt das Abfragen mittels einer Steuereinrichtung (1) der medizintechnischen Behandlungsvorrichtung (100).

In gewissen Aspekten der Erfindung ist das Spezifikationsmerkmal (400) ausgestaltet als Markierung, insbesondere in Form eines Barcodes, eines Dot-Matrix-Codes, eines RFID-Chips oder einer Farbmarkierung oder einer Mischung oder einer Kombination derselben, und/oder weist wenigstens eine der vorstehend genannten Markierungen auf.

In einigen Aspekten der Erfindung ist das Spezifikationsmerkmal (400) vorgesehen zur Befestigung an der medizintechnischen Funktionseinrichtung (300) mittels Aufkleben, Aufdrucken, Beschichten, Einfärben, Durchfärben, Verschnappen, Verrasten und dergleichen.

In manchen Aspekten der Erfindung ist das Spezifikationsmerkmal (400) vorgesehen zur Kennzeichnung eines extrakorporalen Blutschlauchsatzes oder einer bestimmten Ausgestaltung oder Eignung des extrakorporalen Blutschlauchsatzes.

In einigen Aspekten der Erfindung umfasst das Verfahren die Schritte:
- Auswählen einer medizinischen Behandlungsoption an der medizintechnischen Behandlungsvorrichtung (100); und
- In-Kontakt-Bringen und/oder Verbinden einer medizintechnischen Funktionseinrichtung (300) mit der medizintechnischen Behandlungsvorrichtung (100) oder einer Erfassungseinrichtung (200) zum Ermöglichen, das Spezifikationsmerkmal (400) abzufragen, oder mit dieser Absicht.

In einigen Aspekten der Erfindung weist das Verfahren die weiteren Schritte auf:
- Erfassen des Spezifikationsmerkmals (400) mittels der medizintechnischen Behandlungsvorrichtung (100) oder einer hiermit in Signalübertragung verbundenen Erfassungseinrichtung (200);
- Erkennen und/oder Zuordnen einer mit dem Spezifikationsmerkmal (400) verbundenen Funktion.

In gewissen Aspekten umfasst das Verfahren den weiteren Schritt:
- Vergleichen der an der medizintechnischen Behandlungsvorrichtung (100) ausgewählten Behandlungsoption mit der anhand des Spezifikationsmerkmals (400) der medizintechnischen Funktionseinrichtung (300) erkannten Funktion.

In einigen Aspekten umfasst das Verfahren die weiteren Schritte:
- funktionelles Koppeln der medizintechnischen Funktionseinrichtung (300) mit der medizintechnischen Behandlungsvorrichtung (100), wenn die Behandlungsoption und die erkannte Funktion übereinstimmen und/oder miteinander kompatibel sind; und
- Starten definierter Abfragen bezüglich der korrekten funktionellen Ankopplung und/oder Einsetzbarkeit der medizintechnischen Funktionseinrichtung (300) im geplanten medizinischen Behandlungsverfahren.

In bestimmten Aspekten umfasst das Verfahren den weiteren Schritt:
- Ausgeben eines Alarms und/oder einer Anzeige über ein Abweichen zwischen ausgewählter Behandlungsoption und erkannter Funktion, wenn die Behandlungsoption und die erkannte Funktion nicht übereinstimmen und/oder nicht miteinander kompatibel sind.

In manchen Aspekten betrifft die Erfindung eine medizintechnische Funktionseinrichtung (300) mit wenigstens einem wenigstens eine medizintechnische Funktion der medizintechnischen Funktionseinrichtung (300) kennzeichnenden Spezifikationsmerkmal (400), wobei das Spezifikationsmerkmal (400) in, an oder auf der medizintechnischen Funktionseinrichtung (300) angebracht oder vorgesehen und ferner geeignet und/oder konfiguriert ist, um mittels einer mit der medizintechnischen Funktionseinrichtung (300) funktionell gekoppelten oder zu koppelnden medizintechnischen Behandlungsvorrichtung (100) oder einer mit letzterer in Signalübertragung verbundenen oder stehenden Einrichtung abgefragt zu werden.

In bestimmten Aspekten ist die medizintechnische Funktionseinrichtung (300) ausgestaltet zu ihrer Verwendung zu einem extrakorporalen Blutbehandlungsverfahren.

In manchen Aspekten umfasst die medizintechnische Funktionseinrichtung (300) wenigstens einen extrakorporalen Blutschlauchsatz, insbesondere einen in einem dazu ausgelegten und vorgesehenen Organizer (13) angeordneten, extrakorporalen Blutschlauchsatz, oder bestehend hieraus.

In bestimmten Aspekten weist die medizintechnische Funktionseinrichtung (300) ferner wenigstens eine Citrat-Zugabestelle und/oder eine Calcium-Zugabestelle auf.

In manchen Aspekten weist die medizintechnische Funktionseinrichtung (300) ferner wenigstens eine Heparin-Zugabestelle auf.

In einigen Aspekten weist die medizintechnische Behandlungsvorrichtung (100) wenigstens eine Erfassungseinrichtung (200) auf und/oder steht mit dieser in Signalübertragung oder ist mit dieser zur Signalübertragung verbunden, wobei die Erfassungseinrichtung (200) dazu geeignet, vorgesehen und/oder konfiguriert ist, wenigstens ein Spezifikationsmerkmal (400) zu erfassen, welches wenigstens eine Funktion der medizintechnischen Funktionseinrichtung (300) kennzeichnet.

In manchen Aspekten weist die medizintechnische Behandlungsvorrichtung (100) ferner wenigstens eine Erkennungseinrichtung auf, welche dazu geeignet, vorgesehen und/oder konfiguriert ist, anhand des von der Erfassungseinrichtung (200) erfassten Spezifikationsmerkmals eine mit der medizintechnischen Funktionseinrichtung (300) verbundene und/oder dieser zugeordnete Funktion zu erkennen oder zuzuordnen.

In einigen Aspekten weist die medizintechnische Behandlungsvorrichtung (100) ferner wenigstens eine Vergleichseinrichtung auf, welche dazu geeignet, vorgesehen und/oder konfiguriert ist, eine an der medizintechnischen Behandlungsvorrichtung (100) ausgewählte Behandlungsoption mit der anhand des Spezifikationsmerkmals (400) erkannten Funktion der medizintechnischen Funktionseinrichtung (300) zu vergleichen.

In manchen Aspekten weist die medizintechnische Behandlungsvorrichtung (100) ferner wenigstens eine Abfrageeinrichtung auf, welche dazu geeignet, vorgesehen und/oder konfiguriert ist, definierte und/oder vorbestimmte Abfragen bezüglich der korrekten funktionellen Ankopplung und/oder der Einsetzbarkeit der medizintechnischen Funktionseinrichtung (300) in einem geplanten medizinischen Behandlungsverfahren und/oder im Rahmen einer, vor, mit oder nach der Abfrage ausgewählten Behandlungsoption durchzuführen oder deren Durchführung zu veranlassen.

In einigen Aspekten ist die medizintechnische Behandlungsvorrichtung (100) als Blutbehandlungsvorrichtung, insbesondere als Vorrichtung zur Apherese oder Dialyse, wiederum insbesondere zur Hämodialyse, Hämofiltration, Hämodiafiltration, zur Akutdialyse, ausgestaltet.

In manchen Aspekten weist die medizintechnische Behandlungsvorrichtung (100) ferner wenigstens eine Alarmeinrichtung zum Ausgeben eines Alarms und/oder eine Anzeigeeinrichtung auf, welche vorgesehen ist zum Ausgeben eines Alarms oder eine Anzeige bei Nicht-Kompatibilität des Spezifikationsmerkmals (400) bzw. der Funktion der Funktionseinrichtung (300) oder der Funktionseinrichtung (300) selbst und der Behandlungsvorrichtung (100) bzw. der ausgewählten Behandlungsoption.

In einigen Aspekten ist die Steuereinrichtung (1) geeignet und vorgesehen und/oder ausgelegt und/oder konfiguriert zur Durchführung eines hierin offenbarten Verfahrens.

In manchen Aspekten weist die Steuereinrichtung (1) wenigstens eine Erfassungseinrichtung (200) und/oder eine Erkennungseinrichtung und/oder eine Vergleichseinrichtung und/oder eine Abfrageeinrichtung und/oder eine Alarmeinrichtung und/oder eine Anzeigeeinrichtung auf, ist hiermit funktionell gekoppelt und/oder in Signalübertragung verbunden.

In einigen Aspekten umfasst das digitale Speichermedium, insbesondere in Form einer Diskette, CD oder DVD oder EPROM, elektrisch auslesbare Steuersignale, konfiguriert, um derart mit einem programmierbaren Computersystem zusammenwirken, dass die maschinellen Schritte des erfindungsgemäßen Verfahrens veranlasst werden.

In manchen Aspekten umfasst das Computer-Programm-Produkt einen auf einem maschinenlesbaren Träger gespeicherten Programmcode zur Veranlassung der maschinellen Schritte des erfindungsgemäßen Verfahrens, wenn das Computer-Programm-Produkt auf einem Rechner abläuft.

In einigen Aspekten umfasst das Computer-Programm einen Programmcode zur Veranlassung der maschinellen Schritte eines erfindungsgemäßen Verfahrens, wenn das Computer-Programm auf einem Computer abläuft.

Bestimmte erfindungsgemäße Ausführungsformen weisen einen oder mehrere der im Folgenden genannten Vorteile auf.

Die vorliegende Erfindung stellt ein Verfahren zum maschinellen Abfragen eines Spezifikationsmerkmals einer Funktionseinrichtung bereit.

Ein Vorteil der vorliegenden Erfindung besteht in bestimmten Ausführungsformen darin, dass die Auswahl einer medizintechnischen Funktionseinrichtung für ein bestimmtes Behandlungsverfahren ergänzend zur Kontrolle durch den Benutzer kontrolliert werden kann. So kann das Risiko der Folgen eines Irrtums bei der Auswahl der zu verwendenden Funktionseinrichtung durch den Menschen vorteilhaft verringert oder gar ausgeschlossen werden.

In bestimmten Ausführungsformen kann das erfindungsgemäße Verfahren vorteilhaft im Rahmen des Aufrüstens einer AkutDialysemaschine mit einem Disposable-Blutschlauchsatz, der beispielsweise auf einem Organizer vorbereitet ist, eingesetzt werden: Solche Akut-Dialysemaschinen, die zumeist eine Mehrzahl verschiedener Funktionen mit sich entsprechend unterscheidenden Parametern, Behandlungsabläufen und dergleichen ausführen können, werden gerade oftmals in der Notfallmedizin eingesetzt. Sie sollten für diese Verwendung besonders einfach und/oder übersichtlich in ihrer Handhabung sein. Die vorliegende Erfindung stellt ein vorteilhaft einfaches und zuverlässiges Verfahren zum Überwachen der korrekten Aufrüstung der Blutbehandlungsvorrichtung mit der tatsächlich erforderlichen medizintechnischen Funktionseinrichtung bereit.

So kann es erfindungsgemäß vorteilhaft möglich sein, auch bei Verwenden einer - speziell für Notfälle - besonders kompakt ausgestalteten und/oder viele verschiedene Funktionen in sich vereinenden Funktionseinrichtung bzw. einer Funktionseinrichtung mit hohem Integrationsgrad, eine hohe Sicherheit dafür zu gewährleisten, dass die Funktionseinrichtung korrekt verwendet wird. Dies kommt wiederum der Sicherheit des Patienten zugute.

Die vorliegende Erfindung stellt in bestimmten Ausführungsformen vorteilhaft ein einfaches und zuverlässiges Verfahren bereit, um eine sicherheitsrelevante automatische Unterscheidung von Funktionseinrichtungen zu ermöglichen, deren versehentliche Verwechslung für das folgende Behandlungsverfahrens mit beachtlichen Risiken verbunden sein kann. Beispielsweise können im Rahmen einer Blutbehandlung zwei (oder auch deutlich mehr) grundlegende Typen von Kassetten oder Organizer eingesetzt werden, die sich durch die vorgesehenen Schlauchsatzelemente für das vorgesehene Antikoagulationsverfahren unterscheiden. Diese Funktionseinrichtungen weisen gewöhnlich für das jeweilige Antikoagulant (z. B. Heparin oder Citrat/Calcium) spezifische Stichleitungen oder anders ausgestaltete Zugabestellen auf, die einer besonderen Beachtung vor Behandlungsbeginn bedürfen. Erfindungsgemäß ist es vorteilhaft möglich, ein versehentliches Verwenden eines unpassenden Blutschlauchsatzes mittels der erfindungsgemäß vorgesehenen Abfrage des Spezifikationsmerkmals zu verhindern.

Nach Erkennung des Spezifikationsmerkmals der Funktionseinrichtung können vorteilhaft für die konkrete Funktionseinrichtung spezifische Sicherheitsaspekte zuverlässig abgefragt werden.

So ist es mittels der vorliegenden Erfindung in bestimmten Ausführungsformen vorteilhaft möglich, das Vorhandensein bestimmter Leitungen eines Blutschlauchsets funktionsgemäß anhand des Spezifikationsmerkmals zu erfassen. Bei der Kenntnis ihrer Existenz kann beispielsweise deren versehentliches Nicht-Verschließen, was vielleicht aus Sicherheitsgründen allerdings zwingend geboten wäre, mittels Sicherheitsabfrage anzumahnen und damit zu verhindern.

So kann es beim Aufrüsten einer Dialysemaschine besonders wichtig sein, alle Stichleitungen entweder durch Klemmen zu verschließen oder z. B. an entsprechende Lösungsbeutel anzuschließen, so dass deren Enden gegen die Umgebung verschlossen sind. Andernfalls bestünde die Gefahr, dass durch versehentlich offen gebliebene Stichleitungen, Zulaufklemmen oder dergleichen Umgebungsluft in den extrakorporalen Blutkreislauf angesaugt werden könnte oder Blut aus dem extrakorporalen Blutkreislauf in die Umgebung gelangen könnte. Eine solche Gefahr kann insbesondere bei Blutschlauchsätzen mit Citrat-Calcium-Zugabestellen bestehen, da die Citratleitung beispielsweise auf der Saugseite der Blutpumpe angeordnet ist, so dass über ein "offenes" Ende dieser Leitung Luft aus der Umgebung angesaugt werden könnte. Selbst in der venösen Leitung kann aufgrund der Herztätigkeit Unterdruck gegenüber der Umgebung entstehen, so dass beispielsweise über eine dort angeordnete "offene" Calcium-Stichleitung Luft aus der Umgebung angesaugt werden könnte.

Mittels des erfindungsgemäßen Verfahrens kann es in bestimmten Ausführungsformen vorteilhaft möglich sein, eine fehlerhafte Verbindung und/oder Verwendung einer Funktionseinrichtung zu verhindern: Prinzipiell ist es möglich, Schlauchsätze mit Calcium- und Citrat-Leitungen versehentlich für ein Antikoagulationsverfahren einzusetzen, welches nur mit einer Antikoagulanz mittels Heparin arbeitet. Calcium- und Citrat-Leitungen, die bei einem Heparin-Schlauchsatz allerdings nicht vorhanden sind, würden jedoch bei einem versehentlichen Einlegen einer Kassette mit Calcium- und Citrat-Zugabestelle im Rahmen einer Behandlungsoption mit Heparin-Zugabe versehentlich unbemerkt "offen" bleiben, da die Maschinensteuerung für die gewählte Behandlungsoption "Heparin-Zugabe" keine Calcium- und Citrat-Leitungen erwarten und keine Sicherheitsabfragen für eine Behandlungsoption mit Heparin-Zugabe vornehmen würde. Aufgrund der anhand des Spezifikationsmerkmals erkannten Funktion der Funktionseinrichtung ist es erfindungsgemäß jedoch vorteilhaft möglich, ein solches Versehen von vorneherein auszuschließen. Damit wird einer Gefahr für den Patienten und/oder einer Kontaminationsgefahr für die Umgebung vorteilhaft vorgebeugt.

In bestimmten Ausführungsformen der vorliegenden Erfindung kann eine Farbmarkierung vorteilhaft besonders kostengünstig zur Kennzeichnung der Funktionseinrichtung eingesetzt werden.

Neben der Möglichkeit, kostengünstige RGB-Sensoren einsetzen zu können, kann es bei Verwenden einer Farbmarkierung ferner vorteilhaft möglich sein, bereits durch einen Blick auf die Farbe feststellen zu können, ob ein passender Organizer oder Blutschlauch (bzw. eine geeignete Funktionseinrichtung allgemein) ausgewählt wurde. Dadurch ergibt sich in bestimmten Ausführungsformen vorteilhaft eine zusätzliche Kontrollmöglichkeit durch den Benutzer.

In bestimmten Ausführungsformen kann eine an die Farbgebung oder Markierung der Funktionseinrichtung angepasste Farbgebung oder anderweitige Markierung der Verpackung und/oder des Kartons einer Funktionseinrichtung weiter vorteilhaft einfach zu einer korrekten Auswahl der Funktionseinrichtung beitragen.

Das Erfassen des Spezifikationsmerkmals unter Einsatz eines Farbunterscheidungsverfahrens kann gegenüber einer reinen Intensitätsmessung verschiedene Vorteile aufweisen. Die Vorteile umfassen eine erhöhte Sicherheit gegen Störlicht, eine verbesserte Ausfallerkennung, einen größeren Funktionsumfang in Form der Unterscheidung einer Mehrzahl verschiedener Funktionseinrichtungen (die als Disposables ausgestaltet sein können), und dergleichen. Zudem kann der apparative Aufbau weniger aufwendig sein und/oder stabiler arbeiten.

Im Folgenden wird die vorliegende Erfindung unter Bezugnahme auf die beigefügten Figuren rein exemplarisch beschrieben. In den Figuren bezeichnen identische Bezugszeichen gleiche oder identische Elemente. Es gilt:
- Fig. 1: zeigt ein Ablaufdiagramm eines erfindungsgemäßen Verfahrens;
- Fig. 2: zeigt schematisch eine Signalübertragungsverbindung zwischen einer erfindungsgemäßen Behandlungsvorrichtung, einer Erfassungseinrichtung und einer Funktionseinrichtung;
- Fig. 3: zeigt in perspektivischer Ansicht Komponenten einer Erfassungseinrichtung;
- Fig. 4: zeigt eine Vorderansicht eines beispielhaften Spezifikationsmerkmals;
- Fig. 5A: zeigt eine Anordnung der Erfassungseinrichtung an einer erfindungsgemäßen Behandlungsvorrichtung;
- Fig. 5B: zeigt eine erfindungsgemäße Behandlungsvorrichtung, an welcher eine erfindungsgemäße Funktionseinrichtung angeordnet ist;
- Fig. 6: zeigt ein Grundprinzip des Aufbaus und der Funktionsweise der Erfassungseinrichtung;
- Fig. 7: zeigt schematisch eine Anordnung einer Funktionseinrichtung zum Durchführen eines Blutbehandlungsverfahrens unter Zugabe von Citrat und Calcium (gelbe Kassettenmarkierung);
- Fig. 8: zeigt schematisch eine Anordnung einer Funktionseinrichtung zum Durchführen eines Blutbehandlungsverfahrens unter Zugabe von Heparin (blaue Kassettenmarkierung);
- Fig. 9: zeigt schematisch ein Schlauchset mit Heparin-Zugabestelle;
- Fig. 10: zeigt schematisch ein Schaubild einer Anordnung zur Durchführung eines Blutbehandlungsverfahrens unter Zugabe von Heparin;
- Fig. 11: zeigt schematisch ein Schlauchset mit Calcium- und Citrat-Zugabestellen;
- Fig. 12: zeigt schematisch ein Schaubild einer Anordnung zur Durchführung eines Blutbehandlungsverfahrens unter Zugabe von Calcium und Citrat;
- Fig. 13: zeigt schematisch eine Zugangsleitung für eine Heparinbehandlung;
- Fig. 14: zeigt schematisch eine Zugangsleitung für eine Calcium-und-Citrat-Behandlung;
- Fig. 15: zeigt schematisch eine Rückgabeleitung für eine Calcium-und-Citrat-Behandlung;
- Fig. 16A: zeigt schematisch eine erste Kassettenart mit Komponenten eines Citrat-Calcium-Schlauchsets;
- Fig. 16B: zeigt schematisch eine zweite Kassettenart ohne Komponenten eines Citrat-Calcium-Schlauchsets;
- Fig. 17: zeigt schematisch eine Maschinenfront einer erfindungsgemäßen Behandlungsvorrichtung;
- Fig. 18: zeigt schematisch eine Möglichkeit der Positionierung einer Funktionseinrichtung an der Behandlungsvorrichtung;
- Fig. 19: zeigt schematisch das Einlegen von Pumpensegmenten einer Funktionseinrichtung an der Behandlungsvorrichtung;
- Fig. 20: zeigt schematisch das Einlegen von Tropfkammern einer Funktionseinrichtung an der Behandlungsvorrichtung; und
- Fig. 21: zeigt schematisch die Erfassung des Spezifikationsmerkmals der Funktionseinrichtung mittels der erfindungsgemäßen Behandlungsvorrichtung bzw. der Erfassungseinrichtung derselben.
Die Pfeile oder Pfeilspitzen geben jeweils die Strömungsrichtung der entsprechenden Fluide an.

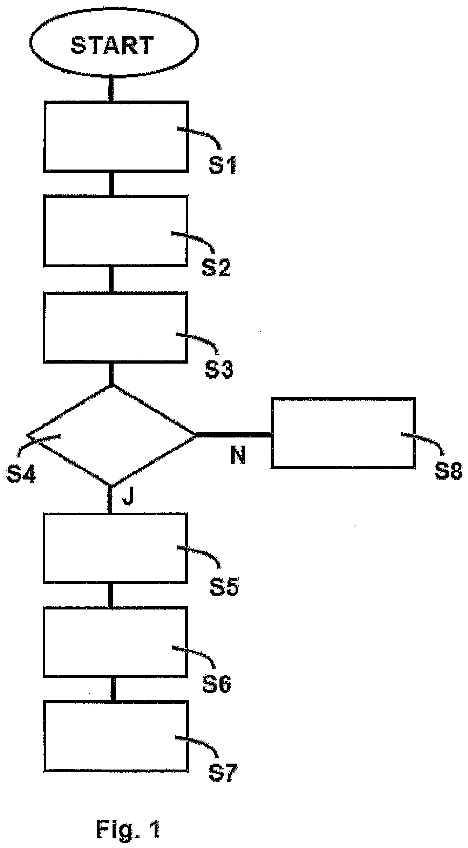

**Fig. 1** zeigt ein Ablaufdiagramm eines erfindungsgemäßen Verfahrens zum Abfragen eines Spezifikationsmerkmals mittels einer Behandlungsvorrichtung oder einer hiermit in Signalübertragung stehenden Einrichtung.

Zunächst wird eine medizinische Behandlungsoption an einer medizintechnischen Behandlungsvorrichtung ausgewählt (Schritt S1).

Eine medizintechnische Funktionseinrichtung, in, auf oder an welcher das Spezifikationsmerkmal angeordnet ist, wird mit der medizintechnischen Behandlungsvorrichtung oder einer Erfassungseinrichtung in Kontakt gebracht und/oder verbunden (Schritt S2). Dabei bleibt es sich gleich, ob Schritt S1 vor den Schritten S2, S3, S4 oder S5 erfolgt, währenddessen, oder anschließend. Schritt S1 kann selbst nach Schritt S6 noch erfolgen, dies kann im Einzelfall jedoch mit unnötiger Arbeit einhergehen.

Das Spezifikationsmerkmal wird mittels der medizintechnischen Behandlungsvorrichtung oder einer hierzu externen Erfassungseinrichtung erfasst (Schritt S3).

Anschließend wird eine mit dem Spezifikationsmerkmal verbundene Funktion erkannt und/oder zugeordnet (Schritt S4).

Nun wird die in Schritt S1 an der medizintechnischen Behandlungsvorrichtung ausgewählte Behandlungsoption mit der in Schritt S4 anhand des Spezifikationsmerkmals der medizintechnischen Funktionseinrichtung erkannten Funktion verglichen (Schritt S5).

Wenn die in Schritt S1 an der medizintechnischen Behandlungsvorrichtung ausgewählte Behandlungsoption und die in Schritt S4 anhand des Spezifikationsmerkmals der medizintechnischen Funktionseinrichtung erkannte Funktion übereinstimmen bzw. miteinander kompatibel sind ("J" für JA), werden die medizintechnische Funktionseinrichtung und die medizintechnische Behandlungsvorrichtung miteinander funktionell gekoppelt (Schritt S6). Diese Kopplung kann ein, insbesondere abschließendes, Aufrüsten der Behandlungsvorrichtung mit der Funktionseinrichtung sein.

Sodann werden definierte Abfragen bezüglich der korrekten funktionellen Ankopplung der medizintechnischen Funktionseinrichtung zum Zweck des geplanten medizinischen Behandlungsverfahren oder Abfragen bezüglich Sicherheitsaspekte gestartet (Schritt S7).

Wenn die an der medizintechnischen Behandlungsvorrichtung ausgewählte Behandlungsoption und die anhand des Spezifikationsmerkmals der medizintechnischen Funktionseinrichtung erkannte Funktion nicht übereinstimmen bzw. nicht miteinander kompatibel sind ("N" für NEIN), so wird ein Alarm und/oder eine Anzeige über ein Abweichen zwischen der ausgewählten Behandlungsoption und der hiervon abweichenden, erkannten Funktion oder Eignung der Funktionseinrichtung ausgegeben.

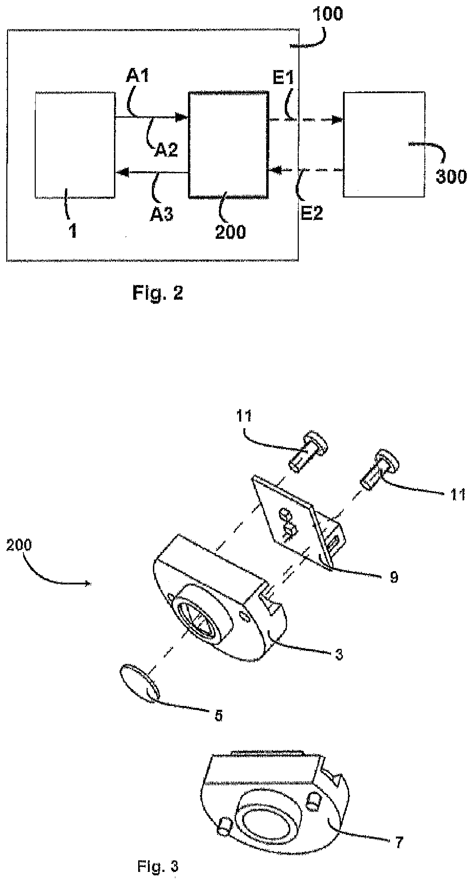

**Fig. 2** zeigt schematisch eine Signalübertragungsverbindung, erzeugt zwischen einer erfindungsgemäßen Behandlungsvorrichtung 100, einer Erfassungseinrichtung 200 und einer Funktionseinrichtung 300.

Die Behandlungsvorrichtung 100, z. B. eine Akutdialysemaschine, weist eine Steuereinrichtung 1 (Geräte-Hardware) auf.

Die Steuereinrichtung 1 fungiert, wie in Fig. 2 gezeigt, als Schnittstelle zwischen der Behandlungsvorrichtung 100 und der Erfassungseinrichtung 200.

Über die Steuereinrichtung 1 werden Steuersignale der Software der Behandlungsvorrichtung 100 an die Erfassungseinrichtung 200 weitergegeben (Signal "A1"). Die Erfassungseinrichtung 200 wird, wie in Fig. 2 gezeigt, über die Steuereinrichtung 1 der Behandlungsvorrichtung mit Energie/Strom versorgt (Signal "A2").

Die Erfassungseinrichtung 200 emittiert zum Erfassen des Spezifikationsmerkmals der Funktionseinrichtung 300 ein Signal E1. Die von der Funktionseinrichtung 300 hierauf hin abgegebenen Informationen werden als Signal E2 an die Erfassungseinrichtung 200 und sodann als Signal "A3" zur Steuereinrichtung 1 der Behandlungsvorrichtung 100 zurückgegeben.

**Fig. 3** zeigt in perspektivischer Ansicht Komponenten einer Erfassungseinrichtung 200.

In Fig. 3 ist die Erfassungseinrichtung 200 beispielhaft als Sensor, z. B. als Farbsensor, gezeigt.

Die Erfassungseinrichtung 200 weist ein Gehäuse 3 auf. In das Gehäuse 3 ist eine Frontscheibe 5 einsetzbar. Das Gehäuse 3 und die Frontscheibe 5 sind mittels einer Abdeckung 7 abdeckbar.

Im zusammengebauten Zustand der Erfassungseinrichtung 200 ist, wie aus der schematischen Anordnung der Komponenten der Fig. 3 zueinander zu erkennen ist, eine Platine 9, z. B. mit einer LED (lichtemittierende Diode, Leuchtdiode) und/oder einem Licht-Frequenz-Wandler, angeordnet.

Mittels Befestigungsschrauben 11 wird die Erfassungseinrichtung 200 an einer Behandlungsvorrichtung (in Fig. 3 nicht gezeigt), z. B. einer Maschinenfront der Behandlungsvorrichtung, verschraubt.

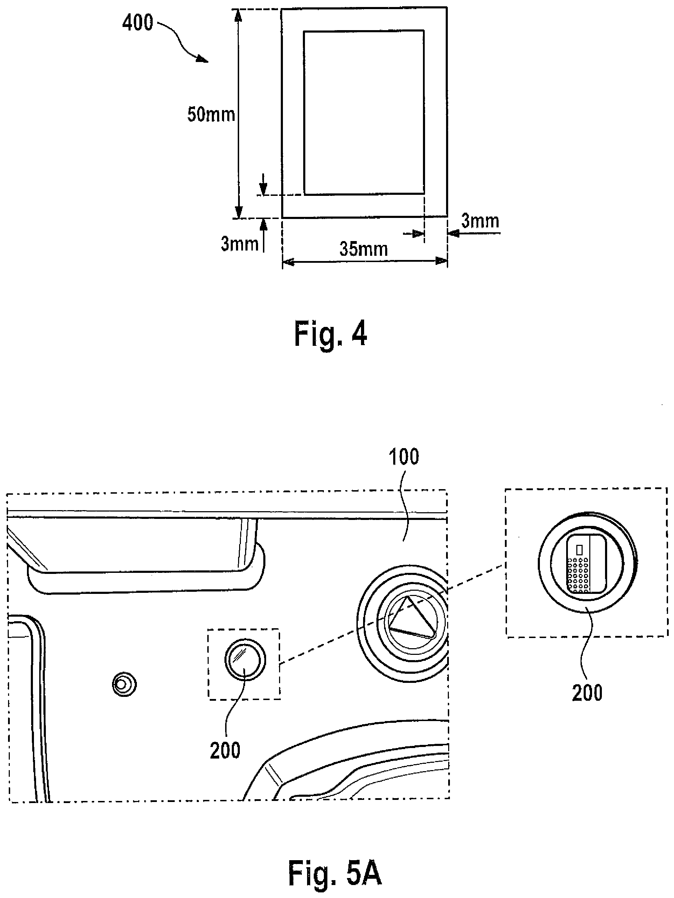

**Fig. 4** zeigt eine Vorderansicht eines beispielhaften Spezifikationsmerkmals 400.

Das Spezifikationsmerkmal 400 wird, beispielsweise mittels Kleben, an der Funktionseinrichtung, etwa einem Organizer oder "Tray", befestigt.

Wie in Fig. 4 gezeigt, wird zur ausreichenden Haftung des Spezifikationsmerkmals 400 (z. B. einer Farbmarkierung) an einer Funktionseinrichtung (in Fig. 4 nicht gezeigt) in dieser Ausführungsform eine Breite der Klebefläche auf allen Seiten mit exemplarischen 3 mm veranschlagt. Unter Berücksichtigung der Lagetoleranz des Trays von beispielsweise ± 5 mm, verbleibt, wie beispielhaft in Fig. 4 gezeigt, noch immer eine ausreichend große auslesbare Fläche des Spezifikationsmerkmals.

Die Maximalmaße eines Spezifikationsmerkmals 400 an einer Funktionseinrichtung können von mehreren Faktoren abhängig sein, beispielsweise der Position der Erfassungseinrichtung an der Behandlungsvorrichtung und dergleichen. In Fig. 4 betragen die Maximalmaße 50 mm x 35 mm.

Farbmarkierungen auf Organizern, Kassetten oder dergleichen können auf unterschiedliche Weise gefertigt werden, wobei vorzugsweise mögliche Farbveränderungen durch die Sterilisierung und/oder Alterung minimiert oder antizipiert werden. Beispiele zum Befestigen eines Spezifikationsmerkmals 400 in, an oder auf einer Funktionseinrichtung schließen eine Befestigung durch selbstklebende Aufkleber, durch Aufdrucken, Beschichten, Einfärben, Durchfärben, Aufbringen, z. B. Verschnappen oder Verrasten einer Farbplakette usw. ein.

**Fig. 5A** zeigt eine an einer erfindungsgemäßen Behandlungsvorrichtung 100 angeordnete Erfassungseinrichtung 200, beispielsweise einen Sensor. Rechts neben der Anordnung ist die Erfassungseinrichtung 200 vergrößert dargestellt.

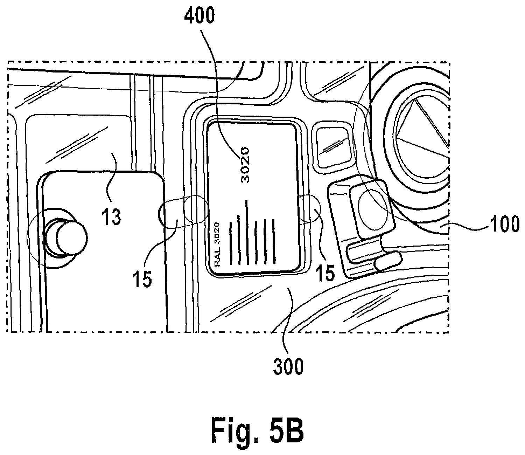

**Fig. 5B** zeigt eine erfindungsgemäße Behandlungsvorrichtung 100, an welcher eine erfindungsgemäße Funktionseinrichtung 300 angeordnet ist.

Die Funktionseinrichtung 300 weist, wie in Fig. 5B gezeigt, ein Tray bzw. einen Organizer 13 auf. Der Organzier 13 trägt in manchen Ausführungsformen vorteilhaft dazu bei, dass die Einzelteile, z. B. Leitungen, Schläuche, Klemmen, usw., in die richtige Position zur Behandlungsvorrichtung 100 gebracht werden können.

Eine Anordnung einzelner Bestandteile wie beispielsweise eines extrakorporalen Blutschlauchsatzes oder Schlauchsets in einem Tray oder Organizer 13 kann in bestimmten Ausführungsformen eine Handhabung des extrakorporalen Blutschlauchsatzes vorteilhaft erleichtern.

An bzw. auf dem Organizer 13, z. B. auf der Rückseite des Organizers 13, ist ein Spezifikationsmerkmal 400, z. B. eine Farbmarkierung, angebracht.

Das Spezifikationsmerkmal 400 wird durch seine konkrete Ausgestaltung auf dem den Organizer 13 in Abstimmung mit der Anordnung der Erfassungseinrichtung zur Behandlungsvorrichtung zum Zwecke des Erfassens geeignet vor der Erfassungseinrichtung 200 positioniert.

Um Schwankungen des Abstands zwischen dem Spezifikationsmerkmal 400 und der Erfassungseinrichtung (in Fig. 5B nicht zu sehen, da das Spezifikationsmerkmal 400 diese verdeckt) über eine Reihe von mit der Blutbehandlungsvorrichtung möglicherweise zu verwendenden unterschiedlichen Organizern zu verhindern, sind Abstandsringe 15 vorgesehen.

Die Abstandsringe 15 können beispielsweise je 0,8 mm dick sein.

Der Organizer 13 bzw. das "Tray" mit aufgerüstetem Blutschlauchsatz - auch als Kassette bezeichnet - ist in manchen Ausführungsformen dazu vorgesehen, steril verpackt vertrieben zu werden.

Der Organizer 13 kann mit unterschiedlichen Blutschlauchsätzen aufgerüstet sein, beispielsweise mit Schlauchsatzelementen für unterschiedliche Antikoagulationsverfahren, z. B. Heparin-Antikoagulation und/oder Citrat-/Calcium-Antikoagulation ("CiCa").

Solche Antikoagulationsverfahren erfordern in bestimmten Ausführungsformen spezielle Stichleitungen am Blutschlauchsatz, die an spezifischen Stellen in den extrakorporalen Blutkreislauf münden, so dass während der Behandlung dem Blut Antikoagulantien zudosiert werden können. Bei Blutschlauchsätzen für die CiCa-Antikoagulation kann zusätzlich auch eine Heparin-Antikoagulation vorgesehen sein, so dass in manchen Ausführungsformen Stichleitungen für beide Antikoagulationsverfahren vorgesehen sein können.

Falls nach Vorgabe einer ausgewählten Behandlungsoption mit Citrat-Calcium-Antikoagulation oder mit Heparin-Antikoagulation weder eine hier beispielhaft erwartete gelbe noch eine blaue Farbmarkierung erkannt wird, so kann eine Fehlermeldung ("unpassende Kassette oder Organizer" oder "Problem mit der Farberkennung von Kassette oder Organizer" o. ä.) ausgegeben werden. Auf diese Weise kann die Durchführung der Behandlung mit einem unpassenden Organizer/Kassette verhindert werden.

Die Blutbehandlungsvorrichtung erkennt automatisch einen ordnungsgemäßen Fall, bei dem zum einen über die Maschinensteuerung eine Behandlungsoption mit Citrat-Calcium-Antikoagulation vorgewählt wurde und danach eine z. B. gelbe Markierung detektiert wurde. Von der Maschinensteuerung werden daraufhin - optional in Verbindung mit entsprechenden Sensoren an der Einlegestellen der Ankoppelebene - automatisch schrittweise spezifische Sicherheitsabfragen durchgeführt. So wird z. B. sichergestellt, dass die vorhandenen Calcium- und Citrat-Leitungen tatsächlich richtig eingelegt bzw. abgesichert werden.

Bei dem Schlauchsystem, das zur Heparin-Behandlung passt, sind diese Komponenten nicht vorhanden und werden daher von der Maschinensteuerung nicht auf korrektes Einlegen überprüft.

Falls nach Vorgabe einer Behandlungsoption mit Citrat-Calcium-Antikoagulation nicht die erwartete gelbe Markierung, sondern z. B. eine blaue Markierung (blau hier für Heparin) erkannt würde, so kann eine Fehlermeldung ("unpassende Kassette/Organizer" o. ä.) und/oder ein Alarm ausgegeben und die Durchführung der Behandlung mit der unpassenden Kassette/dem unpassenden Organizer verhindert werden.

Falls nach Vorgabe einer Behandlungsoption mit Heparin-Antikoagulation nicht die erwartete z. B. blaue Markierung sondern z. B. eine gelbe Markierung (für Citrat-Calcium) erkannt wird, so wird ein Alarm ausgegeben ("unpassende Kassette/Organizer", "Behandlung kann nicht durchgeführt werden", o.ä.) ausgegeben und die Durchführung der Behandlung mit der unpassenden Kassette/dem unpassenden Organizer verhindert.

Dies kann besonders relevant sein, weil eine herkömmliche Maschinensteuerung aufgrund der Vorgabe der Heparin-Antikoagulation einen Blutschlauchsatz ohne Stichleitungen für eine Citrat-Calcium-Antikoagulation zugrunde legt und deshalb auch keine entsprechenden Sicherheitsabfragen zur Absicherung eventuell offener Citrat- und/oder Calcium-Leitungen vorsehen würde.

Falls in einem weiteren ordnungsgemäßen Fall nach Auswahl einer Behandlungsoption mit Heparin-Antikoagulation die Blutbehandlungsmaschine einen Blutschlauchsatz mit z. B. blauer Markierung erkennt, also ein Blutschlauchsatz, Organizer oder eine Kassette zur reinen Heparin-Antikoagulation ohne Citrat- und Calcium-Leitungen eingelegt worden ist, so werden von der Maschinensteuerung, ggf. in Verbindung mit entsprechenden Sensoren an der Einlegestelle der Ankoppelebene, automatisch schrittweise spezifische Sicherheitsabfragen durchgeführt, wodurch vorteilhaft sichergestellt werden kann, dass alle vorhandenen Komponenten tatsächlich richtig eingelegt bzw. abgesichert werden. Die Steuerung geht dabei aufgrund der Farbgebung von einer Kassette ohne Stichleitungen für eine Citrat-Calcium-Antikoagulation aus und sieht deshalb auch keine entsprechenden Sicherheitsabfragen zur Absicherung offener Citrat- und Calcium-Leitungen vor.

Falls die erkannte Kassette also zu der vorgewählten Behandlungsoption passt, so werden von der Maschinensteuerung, ggf. in Verbindung mit entsprechenden Sensoren an der Einlegestellen, spezifische Sicherheitsabfragen durchgeführt, wodurch sichergestellt wird, dass alle relevanten Leitungen abgefragt und abgesichert werden. Andernfalls kann eine Fehlermeldung und/oder ein Alarm ("unpassende Kassette/Organizer" o. ä.) ausgegeben und/oder die Durchführung der Behandlung mit der ggf. bereits eingelegten, unpassenden Kassette verhindert werden.

In bestimmten Ausführungsformen ist möglich und vorgesehen, dass, obwohl anhand der Markierung nicht das zur ausgewählten Behandlungsoption passende Schlauchsystem erkannt wurde, ein Citrat-Calcium-Behandlungsverfahren von der Behandlungsvorrichtung zugelassen wird, da hierbei auf das korrekte Einlegen der (offenen) Citrat- und Calcium-Leitungen geprüft wird. Die Gefahr, die von diesen Schlauchteilen ausgehen würde, ist somit ausgeschlossen.

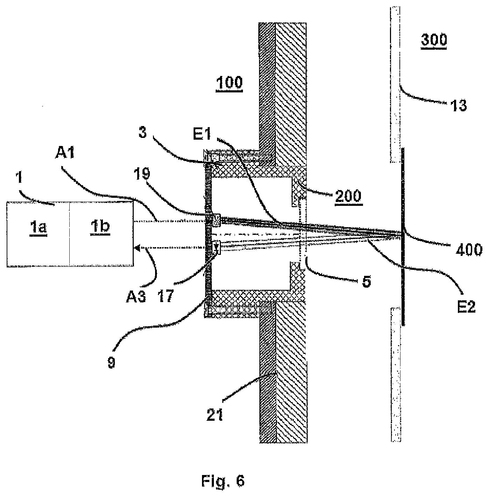

**Fig. 6** zeigt ein Grundprinzip des Aufbaus und der Funktionsweise einer exemplarischen Erfassungseinrichtung 200. In Fig. 6 ist diese ein Farbsensor.

Die Erfassungseinrichtung 200, in Fig. 6 mittig gezeigt, weist in ihrem Gehäuse 3 einen Licht-Frequenz-Wandler 17 und eine RGB-LED (Rot-Grün-Blau-Leuchtdiode) 19 auf.

Licht-Frequenz-Wandler 17 und RGB-LED sind auf einer Platine 9 der Erfassungseinrichtung 200 angeordnet.

Die Erfassungseinrichtung 200 bzw. ihr Gehäuse 3 ist an einer Maschinenoberfläche der Behandlungsvorrichtung 100 angeordnet. Wie in Fig. 6 gezeigt, kann das Gehäuse 3 der Erfassungseinrichtung 200 in ein Gehäuse 21 der Behandlungsvorrichtung 100 eingelassen sein.

Die Frontscheibe 5 der Erfassungseinrichtung 200 ist im Beispiel der Fig. 6 flächenbündig zur Maschinenoberfläche der Behandlungsvorrichtung 100 angeordnet, was den Reinigungsvorgang vorteilhaft erleichtert; bezogen auf die Maschinenoberfläche unterständige oder vorstehende Frontscheiben 5 können jedoch ebenso vorgesehen sein.

In Fig. 6 rechts gezeigt ist eine Funktionseinrichtung 300, welche an bzw. - jeweils bezogen auf ihren Gebrauchszustand - vor der Behandlungsvorrichtung 100 angeordnet ist.

Die Steuereinrichtung 1 der Behandlungsvorrichtung 100 weist eine Softwarekomponente 1a und eine Hardwarekomponente 1b auf.

Zur Erfassung des Spezifikationsmerkmals 400 durch die Behandlungsvorrichtung 100 bzw. die Erfassungseinrichtung 200 derselben wird ein Steuersignal A1 von der Steuereinrichtung 1 an die Erfassungseinrichtung 200 gesendet. Mittels Lichtreflexionsverfahren erfasst die Erfassungseinrichtung 200 das Spezifikationsmerkmal 400 der Funktionseinrichtung 300 und sendet die erhaltenen Informationen als Frequenz A3 an die Steuereinrichtung 1 zurück.

Ein Messverfahren zum Erfassen des Spezifikationsmerkmals 400 kann in bestimmten erfindungsgemäßen Ausführungsformen mittels eines Farbsensors (RGB-Sensor) als Erfassungseinrichtung 200 und einer Farbmarkierung als Spezifikationsmerkmal 400 folgendermaßen ablaufen:
Zur Kalibrierung der Erfassungseinrichtung 200, ggf. bereits als Werkskalibrierung, kann unter Ausblendung von Fremdlicht nacheinander jede LED-Farbe (Rot R, Grün G, Blau B) eingeschaltet und die entsprechende Ausgangsfrequenz des Sensors gemessen werden. Der so erhaltene Frequenzwert kann die Grundfrequenz des Sensors wiederspiegeln, welche bei jedem Messvorgang den eigentlichen Messwert überlagert (Offset).

Zu Beginn des Messvorgangs wird zunächst eine Messung der Frequenz bei ausgeschalteter LED durchgeführt (~ 50 ms). Als nächstes wird die rote LED angeschaltet und gewartet, beispielsweise etwa 0,5 s. Vor einer Messwertübernahme in eine Variable sollte eine Verzögerung von etwa 150 ms abgewartet werden, damit sich LED und Empfänger (Spezifikationsmerkmal) in einem definierten Zustand befinden. Die Frequenz wird nun gemessen (~ 50 ms). Sodann wird die rote LED ausgeschaltet, die grüne LED eingeschaltet und wiederum gewartet (~ 0,5 s). Die Frequenz wird gemessen (~ 50 ms). Nach Ausschalten der grünen LED, wird die blaue LED eingeschaltet und gewartet, beispielsweise wiederum etwa 0,5 s. Die Frequenz wird gemessen (~ 50 ms). Die blaue LED wird ausgeschaltet. Sodann erfolgt eine Wartezeit von etwa 0,5 s. Die Frequenz wird gemessen (~ 50 ms). Es wird erneut ein Fremdlichtwert aufgenommen.

Zur Auswertung der Messung werden zunächst die erste und die letzte Frequenzmessung ohne LED miteinander verglichen (sollte eine Abweichung dieser Werte auftreten, würde der Messvorgang wiederholt werden). Der größere Fremdlichtwert und der kleinere Fremdlichtwert sollten nicht mehr als 20 % voneinander abweichen.

Anschließend werden die resultierenden Frequenzen berechnet. Für jede Farbe werden der bei der Werkskalibrierung bestimmte Offset und der Frequenzwert der ersten Messung ohne LED vom Messwert abgezogen.

Die Faktoren für den Farbabgleich werden berechnet, indem man aus den Offsetwerten den Faktor für Grün aus Rot/Grün und für Blau aus Rot/Blau bildet. Der Faktor für Rot beträgt 1.

Für den Farbabgleich werden die Frequenzen mit den jeweiligen Faktoren multipliziert. Die Erkennung von Farben erfolgt durch den Vergleich von Quotienten von Farbintensitäten (R, G, B) innerhalb vorgegebener Toleranzbereiche.

Es werden die Verhältnisse Rot/Grün, Rot/Blau sowie Grün/Blau berechnet.

Sodann erfolgt eine Abfrage darüber, ob die Verhältnisse größer oder kleiner als 1 sind. Durch Vergleich mit vorgegebenen Werten lassen sich Informationen über die Funktionseinrichtung gewinnen.

Wird beispielsweise eine gelbe Markierung (z. B. für Citrat-Calcium) erkannt, so beträgt das Verhältnis Rot/Grün zum Beispiel > 2,1. Ein Ergebnis der Variable kann hier wahr ("true") sein. Gleichzeitig beträgt das Verhältnis Rot/Blau zum Beispiel > 1,5. Auch hier kann das Ergebnis der Variable wahr ("true") sein. Es wird die Farbe Gelb erkannt.

Wenn als Behandlungsoption ebenfalls ein Verfahren unter Zugabe von Citrat-Calcium ausgewählt wurde, wird eine Übereinstimmung zwischen ausgewählter Behandlungsoption und Funktion oder Eignung der Funktionseinrichtung festgestellt.

Wenn jedoch als Behandlungsoption ein Verfahren unter Zugabe von Heparin ausgewählt wurde, wird keine Übereinstimmung zwischen ausgewählter Behandlungsoption und Funktion der Funktionseinrichtung festgestellt. Es sollte ein Alarm ausgegeben werden.

Beträgt das Verhältnis Rot/Grün zum Beispiel ≤ 2,1, so wird eine blaue Markierung (z. B. für Heparin) erkannt. Ein Ergebnis der Variable kann hier wahr ("true") sein. Gleichzeitig beträgt das Verhältnis Rot/Blau zum Beispiel ≤ 1. Auch hier kann das Ergebnis der Variable wahr ("true") sein. Es wird die Farbe Blau erkannt.

Wenn als Behandlungsoption ebenfalls ein Verfahren unter Zugabe von Heparin ausgewählt wurde, wird eine Übereinstimmung zwischen ausgewählter Behandlungsoption und Funktion der Funktionseinrichtung festgestellt.

Wenn jedoch als Behandlungsoption ein Verfahren unter Zugabe von Citrat und Calcium ausgewählt wurde, wird keine Übereinstimmung zwischen ausgewählter Behandlungsoption und Funktion der Funktionseinrichtung festgestellt. Es sollte wiederum ein Alarm ausgegeben werden.

Sollte noch vor Beginn der Farberkennung eine Fehlermeldung ausgegeben werden, könnte eine Ursachen hierfür sein, dass die Funktionseinrichtung nicht richtig an der Behandlungsvorrichtung positioniert wurde, so dass das Spezifikationsmerkmal nicht von der Erfassungseinrichtung erfasst werden kann. Die Fehlermeldung umfasst optional einen entsprechenden Hinweis.

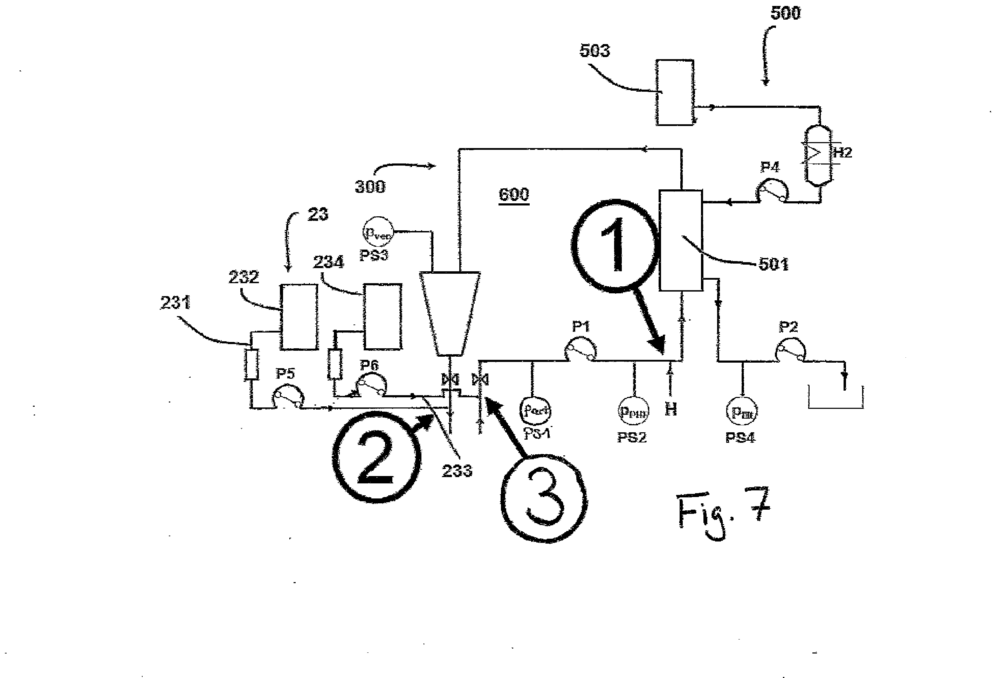

**Fig. 7** zeigt schematisch eine Anordnung einer Funktionseinrichtung 300 zum Durchführen eines Blutbehandlungsverfahrens unter Zugabe von zunächst - oder weiter stromaufwärts - Citrat und später (auf den Fluss eines konkreten, extrakorporal bewegten Erythrozyts bezogen) - oder weiter stromabwärts - Calcium.

Auf Antikoagulationsleitungen für Calcium und Citrat wird auf der maschinenseitigen Ankoppelfläche der Funktionseinrichtung 300 an einer definierten Stelle mit einem Spezifikationsmerkmal, z. B. in Form einer gelben Farbmarkierung, etwa einem gelben Aufkleber, hingewiesen. Dies gilt in bestimmten Ausführungen unabhängig davon, ob zusätzlich auch eine Heparinleitung vorgesehen ist.

Die Funktionseinrichtung 300 stellt einen extrakorporalen Blutkreislauf oder Blutschlauchsatz für eine Blutbehandlung eines Patienten, z. B. eine Hämofiltration, dar.

Auf der in Fig. 7 rechten Seite ist ein Dialysatkreislauf 500 mit einem Dialysator 501 und einer Quelle 503 für Filtrat oder Dialysat gezeigt.

Der mit "600" gekennzeichnete Teil stellt den Blutkreislauf dar. Der Blutkreislauf 600 ist im Wesentlichen oder vollständig Teil der Funktionseinrichtung 300.

Die in Fig. 7 gezeigte Funktionseinrichtung 300 stellt schematisch einen für eine Dialyse (CVV-HD = Continuous Venous to Venous Hemodialysis) vorgesehenen extrakorporalen Blutschlauchsatz dar, der Citrat-Calcium-Schlauchelemente 23 aufweist. Die Citrat-Calcium-Schlauchelemente 23 umfassen eine erste Zuleitung 231 zum Zuführen von Calcium aus einer Quelle 232 für Calcium mittels einer Pumpe P5 sowie eine sich hiervon unterscheidende zweite Zuleitung 233 zum Zuführen von Citrat aus einer Quelle 234 für Citrat mittels einer Pumpe P6.

Die Zuleitungen 231 bzw. 233 münden in den extrakorporalen Blutschlauchsatz an den mit ② bzw. ③ gekennzeichneten Konnektionsstellen, welche jeweils Abzweige des extrakorporalen Blutschlauchsatzes mit offenen Konnektionsstellen darstellen (offen, da im Falle einer Calcium- und Citrat-Zugabe ein Eintreten von Calcium bzw. Citrat an der entsprechenden Stelle in den extrakorporalen Blutschlauchsatz erfolgen soll).

Die Zugabe von Calcium erfolgt, wie in Fig. 7 gezeigt, hinter dem bzw. stromab des Dialysators 501. Sollte die Konnektionsstelle ② versehentlich offen bleiben, bestünde somit die Gefahr, extrakorporal behandeltes Blut an der Konnektionsstelle ② aus dem extrakorporalen Blutschlauchsatz in die Umgebung zu verteilen.

Die Zugabe von Citrat erfolgt, wie in Fig. 7 gezeigt, vor dem bzw. stromauf des Dialysators 501. Sollte die Konnektionsstelle ③ versehentlich offen bleiben, bestünde somit die Gefahr, mittels der Blutpumpe P1 Luft in den extrakorporalen Blutschlauchsatz einzusaugen und damit ggf. in den mit dem extrakorporalen Blutschlauchsatz verbundenen Patienten einzutragen.

In manchen Citrat-Calcium-Kassetten oder -Schlauchsätzen ist zusätzlich eine Zugabestelle für Heparin vorgesehen, in anderen wiederum nicht.

Eine Zugabestelle H für Heparin ist in Fig. 7 als Konnektionsstelle ① gekennzeichnet. Bei einer Kassette für Citrat- und Calcium-Zugabe ist die Konnektionsstelle ① beispielsweise durch ein Rückschlagventil gesichert. Eine potentielle Gefahr aufgrund einer offenen Konnektionsstelle ① kann so vorteilhaft ausgeschlossen werden.

Das Citrat- und Calcium-Schlauchset enthält - im Vergleich zum beispiel zu einem Heparin-Schlauchset - wie beschrieben zusätzliche offene Konnektionsstellen ② und ③, über welche, sollten sie versehentlich nicht geschlossen werden, Blut aus- und Luft eintreten könnte. Diese müssen daher zuverlässig verschlossen werden.

Die Bezeichnungen p(art) oder PS1, p(PHF) oder PS2, p(ven) oder PS3 stehen für Drucksensoren zum Erfassen des Blutdrucks an den entsprechenden Stellen im extrakorporalen Blutschlauchsatz.

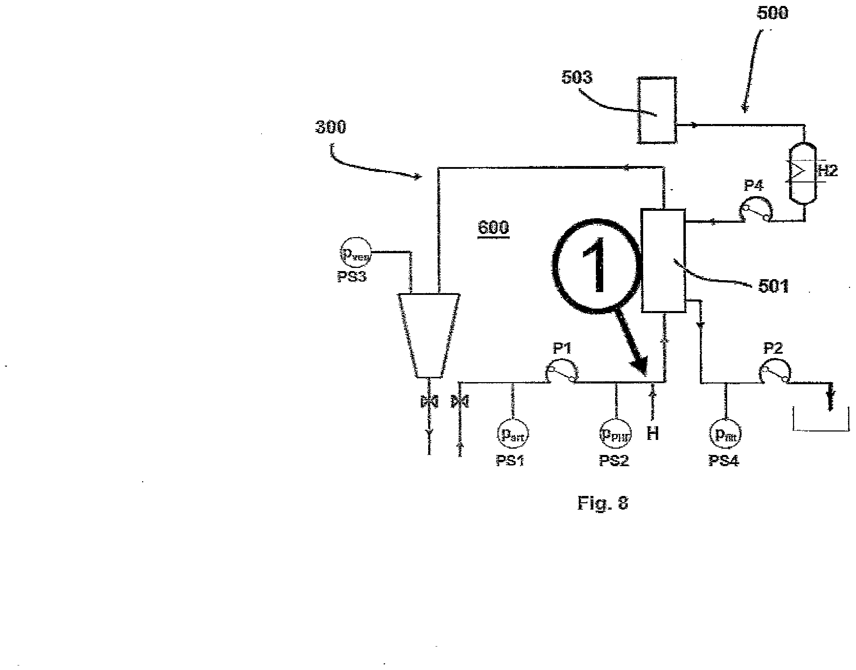

**Fig. 8** zeigt schematisch eine Anordnung einer Funktionseinrichtung 300 zum Durchführen eines Blutbehandlungsverfahrens unter Zugabe von Heparin.

In bestimmten Ausführungsformen erhalten Kassetten für die alleinige Heparin-Antikoagulation zur Unterscheidung gegenüber einer Kassette mit Citrat- und Calcium-Zugabe beispielsweise eine blaue Farbmarkierung, z. B. einen blauen Aufkleber ("Heparin = Blau").

Damit kann das Pflegepersonal vorteilhaft bereits optisch einen ersten Hinweis auf die Eignung der jeweiligen Kassette für bestimmte Behandlungsoptionen erhalten.

Der Aufbau der Funktionseinrichtung 300 in Fig. 8 entspricht im Wesentlichen jenem der in Fig. 7 gezeigten, allerdings liegt hier kein Citrat- und Calcium-Schlauchelement vor. Die Behandlungsvorrichtung, die eine blaue Farbmarkierung erkennt, erwartet ein Set ohne offene Citrat- und Calcium-Konnektionsstellen und würde diese daher auch nicht abfragen.

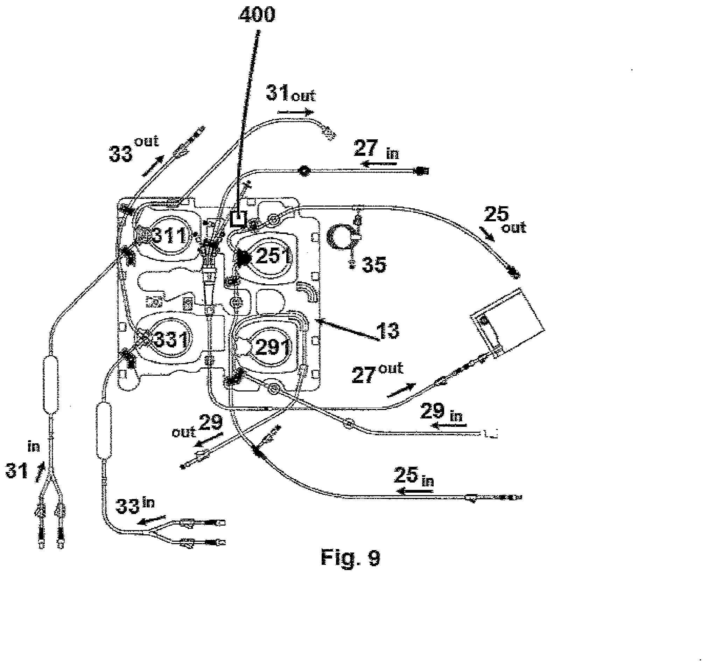

In den folgenden Figuren 9 bis 13 geben die Bezeichnungen "in" und "out" in den Figuren das Einströmen ("in") bzw. Ausströmen ("out") des entsprechenden Fluids in die Funktionseinrichtung hinein bzw. aus der Funktionseinrichtung heraus an.

**Fig. 9** zeigt schematisch ein Schlauchset mit Heparin-Zugabestelle.

Das Heparin-Schlauchset, angeordnet auf einem Organizer 13 und gekennzeichnet durch ein Spezifikationsmerkmal 400, z. B. eine blaue Farbmarkierung, weist eine Zugangsleitung 25in und 25outfür Blut auf, welche vom Patienten zum Filter, z. B. einem Dialysierfilter, führt.- Das Schlauchset weist ferner eine Rückgabeleitung 27in und 27out für Blut auf, welche vom Filter (in Fig. 9 nicht gezeigt) zum Patienten (in Fig. 9 nicht gezeigt) führt. Ferner ist eine Filtratleitung, bezeichnet als 29in und 29out, vorgesehen.

Eine Dialysatleitung 31in und 31out für Dialysat bzw. Dialysierflüssigkeit von einem Lösungsbeutel (in Fig. 9 nicht gezeigt) zum Filter oder Anschlussstück ist ebenfalls vorgesehen. Ebenso ist eine Substituatleitung 33in und 33out für den Transport von Austauschflüssigkeit bzw. Substituat von einem Lösungsbeutel (in Fig. 9 nicht gezeigt) zum Anschlussstück in der Zugangsleitung 25 oder der Rückgabeleitung 29 vorhanden.

Eine Heparin-Zugabestelle 35 ist vorgesehen zur Zugabe von Heparin stromauf des Filters in die Blutschlauchleitung 25out und stromab der Blutpumpe 251.

In Fig. 9 sind ferner eine Filtratpumpe 291, eine Dialysatpumpe 311 und eine Substituatpumpe 331 dargestellt.

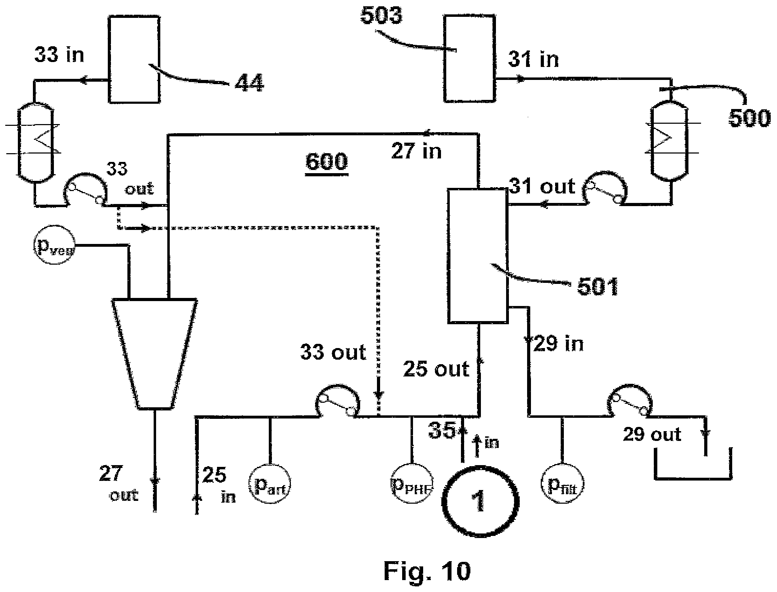

**Fig. 10** zeigt schematisch ein Schaubild einer Anordnung zur Durchführung eines Blutbehandlungsverfahrens unter Zugabe von Heparin unter Verwendung eines Heparin-Schlauchsets, wie in Fig. 9 beschrieben.

In Fig. 10 gezeigt sind Leitungen des extrakorporalen Blutkreislaufs 600 und des Dialysatkreislaufs 500. Substituatflüssigkeit wird aus einer Quelle 44 für Substituatflüssigkeit zugeführt.

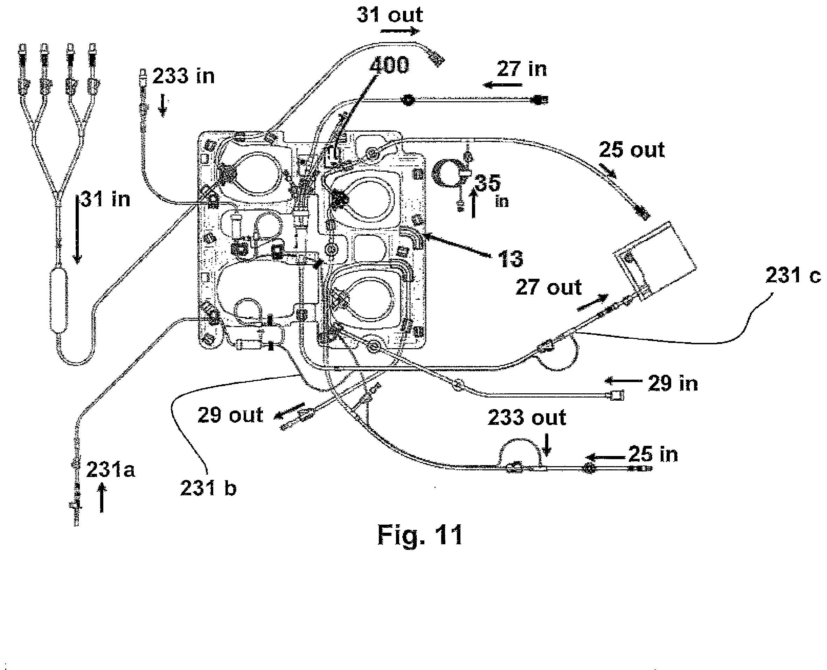

**Fig. 11** zeigt schematisch ein Schlauchset für eine Behandlung unter Citrat-Calcium-Zugabe.

Das Citrat-Calcium-Schlauchset, angeordnet auf einem Organizer 13 und gekennzeichnet durch ein Spezifikationsmerkmal 400, z. B. eine gelbe Farbmarkierung, weist eine Calcium-Leitung 231a für die Zugabe von Calcium auf, welche in einen weiteren Abschnitt 231b übergeht und in einer Calcium-Zugabestelle 231c für die Zugabe von Calcium in die venöse Rückgabeleitung 27out mündet.

Das Citrat-Calcium-Schlauchset weist zudem für die Zugabe einer Citrat-Lösung einen Konnektor 233in auf. Über die zugehörige Leitung gelangt Citrat-Lösung in die z. B. an der mit 233out bezeichneten Stelle in die vom Patienten kommende Zugangsleitung 25in (d. h. in die arterielle Blutleitung).

Das Citrat-Calcium-Schlauchset weist überdies eine Heparin-Zugabestelle 35 auf.

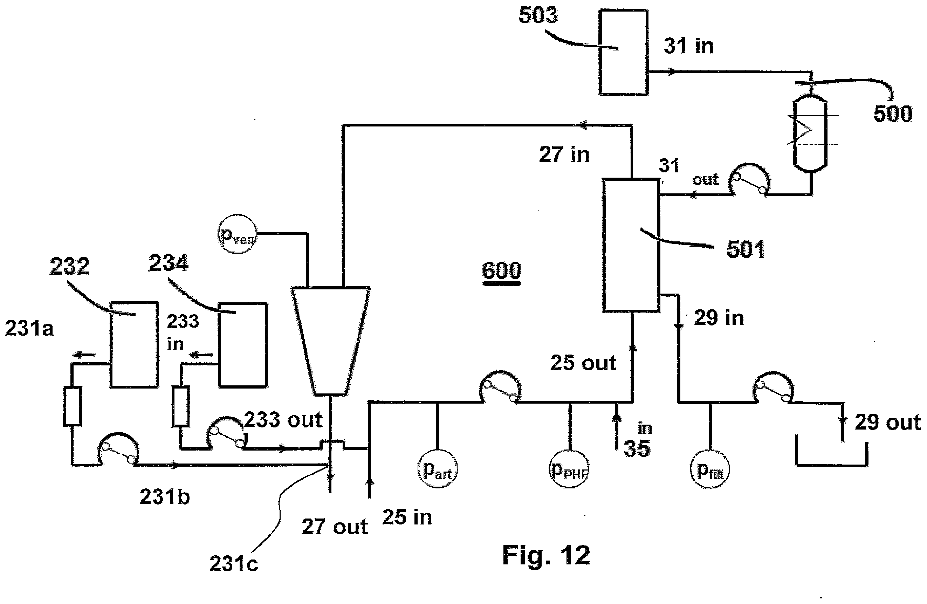

**Fig. 12** zeigt schematisch ein Schaubild einer Anordnung zur Durchführung eines Blutbehandlungsverfahrens unter Zugabe von Citrat sowie Calcium unter Verwendung eines Citrat-Calcium-Schlauchsets, wie in Fig. 11 beschrieben.

In Fig. 12 gezeigt sind Leitungen des extrakorporalen Blutkreislaufs 600 und des Dialysatkreislaufs 500. Calcium wird aus einer Quelle 232 für Calcium zugeführt, Citrat aus einer Quelle 234 für Citrat.

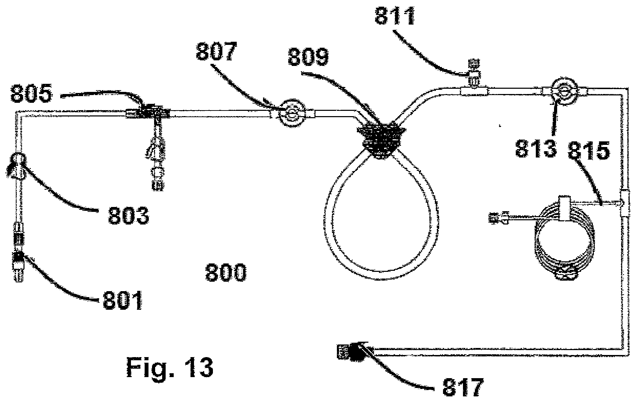

**Fig. 13** zeigt schematisch eine Zugangsleitung 800, welche im Wesentlichen der Zugangsleitung 25 wie z. B. in Fig. 9 gezeigt entspricht, und welche sich eignet für eine Blutbehandlung unter Zugabe von Heparin.

Die Zugangsleitung 800 der Fig. 13 weist einen Patientenkonnektor mit Kappe 801, eine Schlauchklemme 803, einen Infusionsport 805, eine in der Zugangsleitung 800 gelegene Druckmessstelle 807, ein Pumpensegment 809 für eine Rollenpumpe, ein Anschlussstück 811 mit Rückschlagventil für Austauschflüssigkeit, eine Druckmessstelle 813 für einen Vor-Filter-Druck, eine Anschlussleitung 815 für eine Heparin-Spritze sowie einen Filteranschluss 817 mit Kappe auf.

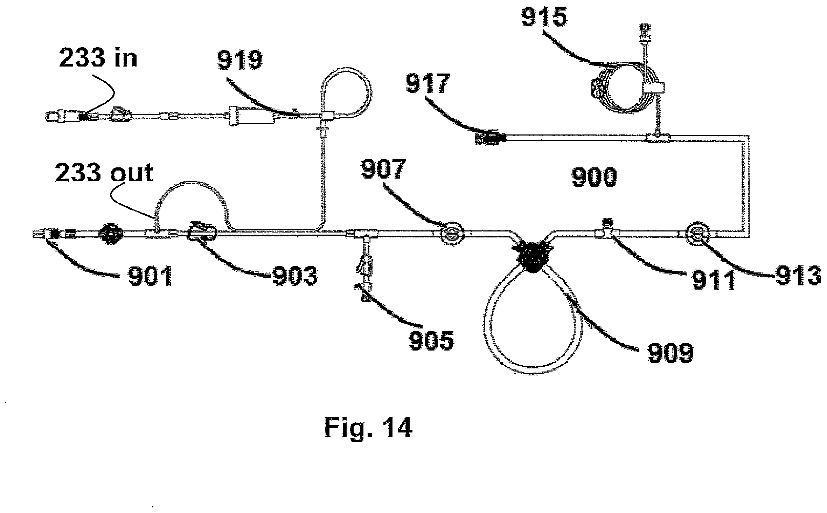

**Fig. 14** zeigt schematisch eine Zugangsleitung 900, welche im Wesentlichen der Zugangsleitung 25 wie z. B. in Fig. 11 gezeigt entspricht, und welche sich eignet für eine Blutbehandlung unter Zugabe sowohl von Heparin als auch von Citrat.

Die Zugangsleitung 900 mit Citratzuleitung 233in und 233out weist, wie in Fig. 14 gezeigt, einen Patientenkonnektor 901 mit Kappe, eine Schlauchklemme 903, einen Infusionsport 905, eine Druckmessstelle 907 in der Zugangsleitung 900, ein Pumpensegment 909 für eine Rollenpumpe, ein Anschlussstück 911 mit Rückschlagventil für Austauschflüssigkeit, eine Druckmessstelle 913 für einen Vor-Filter-Druck, eine Anschlussleitung 915 für eine Heparin-Spritze, einen Filteranschluss 917 mit Kappe sowie eine Citrat-Zuleitung 919 mit Pumpensegment und Tropfkammer auf.

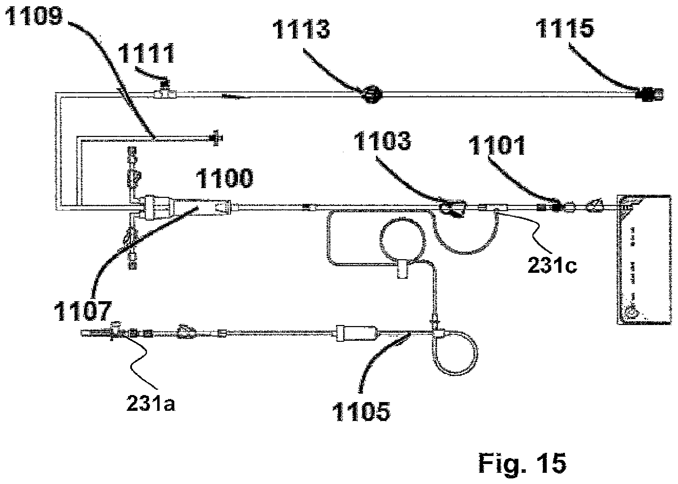

**Fig. 15** zeigt schematisch eine Rückgabeleitung 1100, welche im Wesentlichen der Rückgabeleitung 27 wie z. B. in Fig. 11 gezeigt entspricht, und welche sich eignet für eine Blutbehandlung unter Zugabe von Calcium.

Die Rückgabeleitung 1100 weist, wie in Fig. 15 gezeigt, einen Patientenkonnektor 1101 mit Beutel, eine Schlauchklemme 1103, eine Calcium-Zuleitung 1105 mit Pumpensegment und Tropfkammer, einen venösen Blasenfänger 1107 eine Druckmessleitung 1109 für den Rückgabedruck, ein Anschlussstück 1111 mit Rückschlagventil für Austauschflüssigkeit, eine Probenentnahmestelle 1113 und/oder Zuspritzstelle sowie einen Filteranschluss 1115 mit Kappe auf.

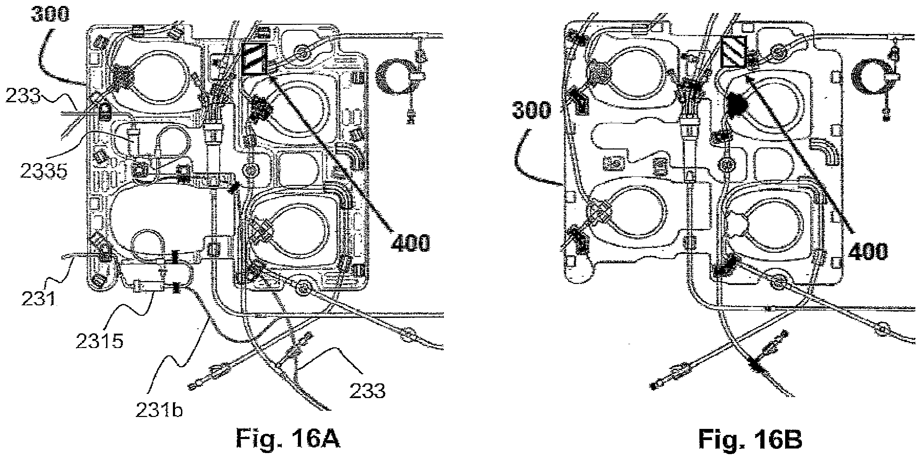

**Fig. 16A** zeigt schematisch eine erste Kassettenart, welche mit Tropfkammern 2335 und 2315 für Calcium- und Citratlösungen sowie Leitungen 231 und 233 zur Zugabe von Citrat und Calcium aufweist und daher als ein Citrat-Calcium-Schlauchset ausgestaltet ist. Die Kassette der Fig. 16A eignet sich auch zur Zugabe von Heparin.

**Fig. 16B** zeigt schematisch eine zweite Kassettenart ohne Komponenten für die Zugabe von Citrat oder Calcium. Die Kassette oder Funktionseinrichtung 300 der Fig. 16B eignet sich nur zur Zugabe von Heparin.

Die Spezifikationsmerkmale 400 der beiden Funktionseinrichtungen unterscheiden sich demnach (z. B. gelb, wenn ein Citrat-Calcium-Schlauchset vorhanden ist, siehe Fig. 16A, und blau, wenn kein Citrat-Calcium-Schlauchset vorhanden ist, siehe Fig. 16B), was hier durch die verschiedene Schraffierung der Spezifikationsmerkmale angedeutet ist.

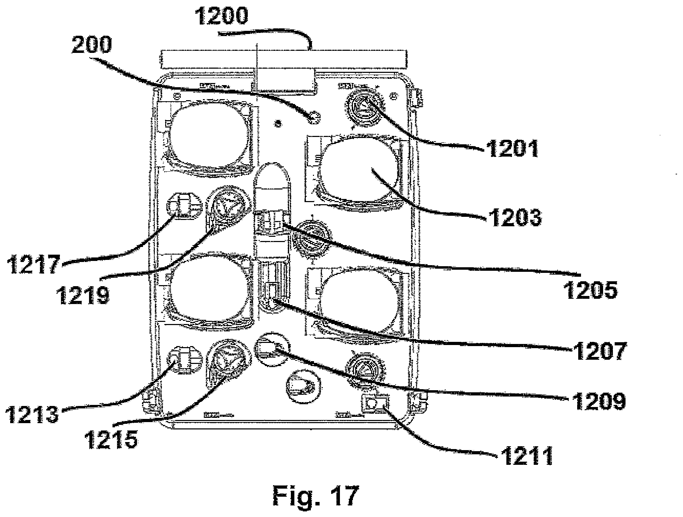

**Fig. 17** zeigt schematisch eine Maschinenfront 1200 einer erfindungsgemäßen Behandlungsvorrichtung.

Die Maschinenfront 1200 weist eine Erfassungseinrichtung 200 zum Erfassen des Spezifikationsmerkmals einer Funktionseinrichtung (in Fig. 17 beides nicht gezeigt) auf.

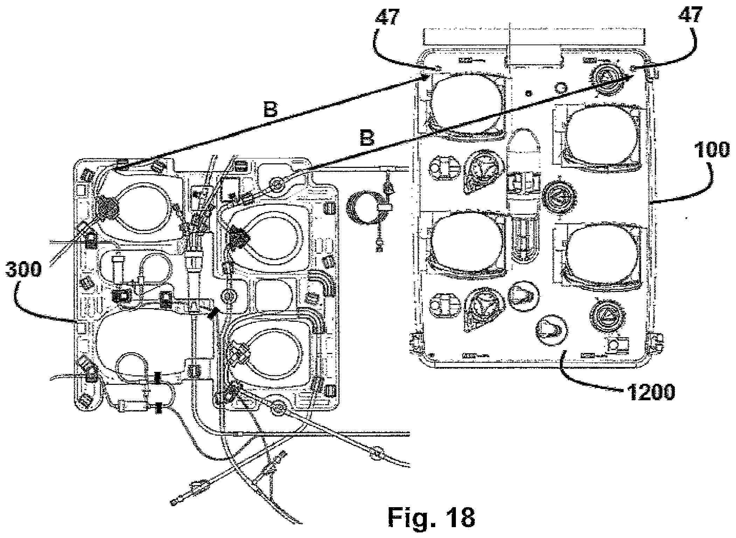

Die Maschinenfront 1200 der Behandlungsvorrichtung weist, wie in Fig. 18 gezeigt, eine Druckmesseinheit 1201, eine Pumpe 1203, einen Halter 1205 für einen venösen Blasenfänger, einen Luftblasendetektor 1207, z. B. in Form eines optischen Detektors, eine Schlauchklemme 1209, einen Blutleckage-Sensor 1211, einen Halter 1213 mit einer Überwachung für eine Calcium-Tropfkammer, eine Calcium-Pumpe 1215 mit einem Einlegeschalter, einen Halter 1217 mit einer Überwachung für eine Citrat-Tropfkammer sowie eine Citrat-Pumpe 1219 mit einem Einlegeschalter auf.

**Fig. 18** zeigt schematisch eine Möglichkeit der Positionierung einer Funktionseinrichtung 300 an der Behandlungsvorrichtung 100.

Zur Befestigung der Funktionseinrichtung 300 an der Behandlungsvorrichtung 100 sind an der Maschinenfront 1200 der Behandlungsvorrichtung 100 Aufhängepins 47 vorgesehen.

Die beiden Blockpfeile B geben die Richtung der Befestigung der Funktionseinrichtung 300 an der Behandlungsvorrichtung 100 an.

Um die Behandlungsvorrichtung 100 mit der Funktionseinrichtung 300 aufzurüsten, wird das Schlauchsystem der Funktionseinrichtung 300 in die entsprechenden Abschnitte der Behandlungsvorrichtung 100 eingelegt und/oder damit verbunden.

Beispielsweise werden beim Aufrüsten einer Akut-Dialysemaschine mit einer Kassette vom Anwender verschiedene Elemente des Blutschlauchsatzes in entsprechende Aufnahmen (z. B. Klemmen, Rollenpumpen, etc.) an der Maschinenfront 1200 "eingelegt". Entsprechend der über die Maschinensteuerung vorgewählten Behandlungsoption wird erfindungsgemäß von der Maschinensteuerung (Steuereinrichtung) - und optional entsprechender Sensorik an der Dialysemaschine - schrittweise überprüft, ob alle Komponenten zu der vorgewählten Behandlungsoption richtig eingelegt sind. Dabei bezieht sich ein Satz von automatischen Sicherheitsabfragen auf die vorgewählte Behandlungsoption und die dazu gehörende, erfasste Kassette mit ihren spezifischen Elementen.

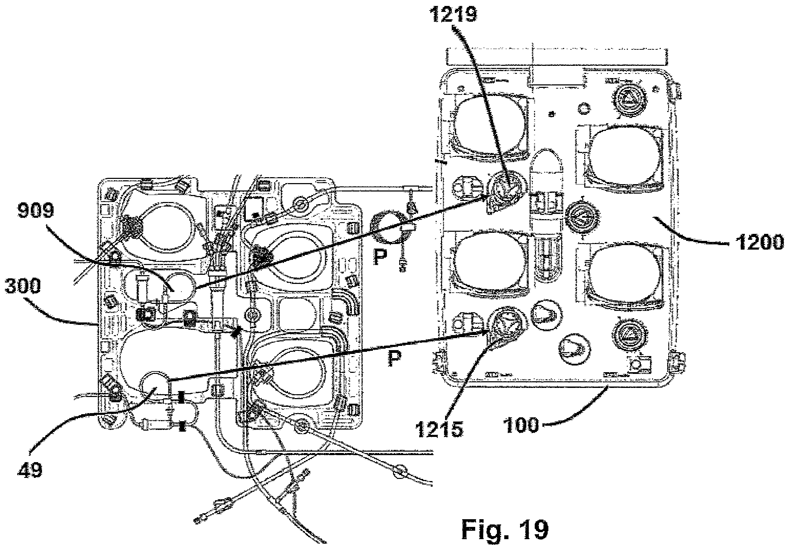

**Fig. 19** zeigt schematisch das Einlegen von Pumpensegmenten, Pumpensegment 909 für die Citrat-Zuleitung und Pumpensegment 49 für die Calcium-Zuleitung, einer Funktionseinrichtung 300 an der Behandlungsvorrichtung 100.

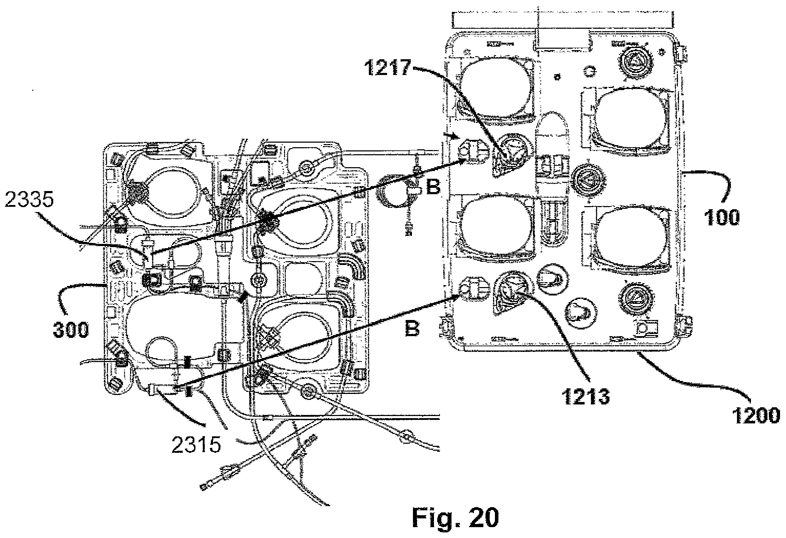

**Fig. 20** zeigt schematisch das Einlegen von Tropfkammern einer Funktionseinrichtung 300 mit Citrat-Calcium-Zugabe an der Behandlungsvorrichtung 100.

Eine Citrat-Tropfkammer 51 der Funktionseinrichtung 300 wird in den Halter 1217 für die Citrat-Tropfkammer der Behandlungsvorrichtung 100 eingelegt; eine Calcium-Tropfkammer 53 der Funktionseinrichtung 300 wird in den Halter 1213 für die Calcium-Tropfkammer an der Behandlungsvorrichtung 100 eingelegt. Die beiden Blockpfeile B geben wiederum die Richtung der Befestigung der Funktionseinrichtung 300 an der Behandlungsvorrichtung 100 an.

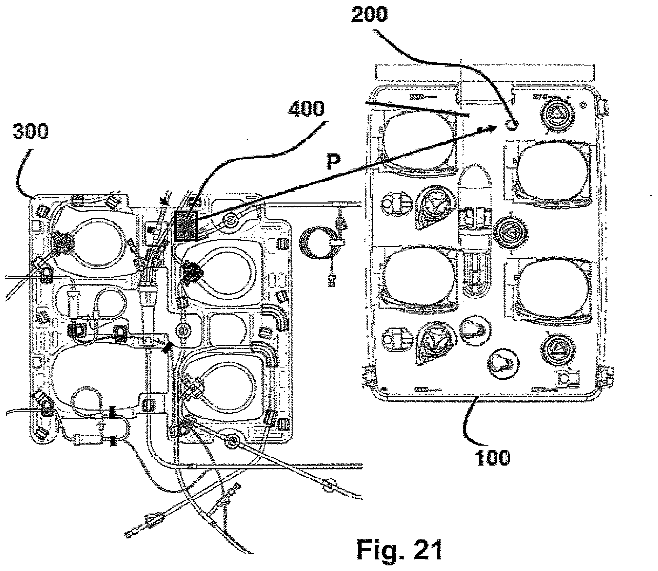

**Fig. 21** zeigt schematisch eine die Detektion der Funktionseinrichtung 300 bzw. ihres Spezifikationsmerkmals 400 mittels der erfindungsgemäßen Behandlungsvorrichtung 100 bzw. der Erfassungseinrichtung 200 derselben.

Der Blockpfeil P veranschaulicht die Positionierung der Funktionseinrichtung 300 bzw. des Spezifikationsmerkmals 400 vor bzw. relativ zur Behandlungsvorrichtung 100 bzw. ihrer Erfassungseinrichtung 200.

Die Blutbehandlungsvorrichtung erkennt beim z. B. manuellen Anbringen der Funktionseinrichtung 300 an der Ankoppelfläche der Behandlungsvorrichtung 100 automatisch über die Erfassungseinrichtung 200, z. B. einen Farberkennungssensor (RGB Sensor), ob eine gelbe oder eine blaue Farbmarkierung (Spezifikationsmerkmal 400; "gelb" z. B. für Zugabe von Citrat-Calcium; "blau" z. B. für Zugabe von Heparin) vorliegt. Je nachdem welche Behandlungsoption vorher an der Behandlungsvorrichtung 100 gewählt wurde, unterscheidet die Maschinensteuerung verschiedene ordnungsgemäße Fälle und mögliche Fehlerfälle (siehe oben).

Da erfindungsgemäß vorteilhaft unterschiedliche Schlauchsysteme speziell für unterschiedliche Arten der Antikoagulation des extrakorporalen Blutkreislaufs durch eine maschinenlesbare Markierung unterscheidbar sind, kann es vorteilhaft möglich sein, durch die speziellen Sicherheitsabfragen, die auf bestimmte Komponenten des erkannten Schlauchsystems zugeschnitten sind, die Gefahr von Blutverlust und/oder Luftinfusion, die von offenen Schlauchenden und nicht eingelegten Schlauchsystemkomponenten ausgeht, zu vermeiden.

### Bezugszeichenliste

| **Bezugszeichen** | **Beschreibung** |
|---|---|
| **100** | Behandlungsvorrichtung |
| **200** | Erfassungseinrichtung |
| **300** | Funktionseinrichtung |
| **400** | Spezifikationsmerkmal |
| **500** | Dialysatkreislauf |
| 501 | Dialysator |
| **503** | Quelle für Dialysat |
| **600** | Blutkreislauf |
| 1 | Steuereinrichtung der Behandlungsvorrichtung |
| 1a | Software |
| 1b | Hardware |
| 3 | Gehäuse der Erfassungseinrichtung |
| 5 | Frontscheibe |
| 7 | Abdeckung |
| 9 | Platine |
| 11 | Befestigungsschrauben |
| 13 | Organizer |
| 15 | Abstandsringe |
| 17 | Licht-Frequenz-Wandler |
| 19 | RGB-LED (Rot-Grün-Blau-Leuchtdiode) |
| 21 | Gehäuse der Behandlungsvorrichtung |
| 23 | Calcium-Citrat-Schlauchelemente |
| 231 | Zuleitung zum Zuführen von Calcium |
| 231a | Calcium-Leitung für Zugabe von Calcium |
| 231b | Calcium-Leitung für Zugabe von Calcium |
| 231c | Calcium-Zugabestelle für Zugabe in die Rückgabeleitung zur Rückgabe von Blut an den Patienten |
| 2315 | Tropfkammer für Calciumlösung |
| 232 | Quelle für Calcium |
| 233 | Zuleitung zum Zuführen von Citrat |
| 233in | Konnektor für Citrat-Lösungsbeutel |
| 233out | Anschlussstelle für die Zugabe von Citrat in die vom Patienten kommende Zugangsleitung für Blut |
| 2335 | Tropfkammer für Citratlösung |
| 234 | Quelle für Citrat |
| 25in | Zugangsleitung (für Blut) vom Patienten |
| 25out | Zugangsleitung (für Blut) zum Filter |
| 251 | Blutpumpe |
| 27in | Rückgabeleitung (für Blut) vom Filter |
| 27out | Rückgabeleitung (für Blut) zum Patienten |
| 29in | Filtratleitung vom Filter |
| 29out | Filtratleitung zum Beutel |
| 291 | Filtratpumpe |
| 31in | Dialysatleitung vom Beutel |
| 31out | Dialysatleitung zum Filter oder Anschlussstück |
| 311 | Dialyatpumpe |
| 33in | Substituatleitung vom Beutel |
| 33out | Substituatleitung zum Anschlussstück in Zugangs- oder Rückgabeleitung |
| 331 | Substituatpumpe |
| 35 | Heparin-Zugabestelle |

| | |
|---|---|
| 44 | Quelle für Substituat |
| 47 | Aufhängepins |
| 49 | Pumpensegment für die Calcium-Zuleitung |
| **800** | Zugangsleitung zur Behandlung unter Zugabe von Heparin |
| 801 | Patientenkonnektor |
| 803 | Schlauchklemme |
| 805 | Infusionsport |
| 807 | Druckmessstelle in der Zuleitung 800 |
| 809 | Pumpensegment |
| 811 | Anschlussstück für Austauschflüssigkeit |
| 813 | Druckmessstelle für Vor-Filter-Druck |
| 815 | Anschlussstelle für Heparin-Spritze |
| 817 | Filteranschluss |
| **900** | Zugangsleitung mit Citratzuleitung |
| 901 | Patientenkonnektor |
| 903 | Schlauchklemme |
| 905 | Infusionsport |
| 907 | Druckmessstelle in der Zugangsleitung 800 |
| 909 | Pumpensegment für die Citratzuleitung |
| 911 | Anschlussstück für Austauschflüssigkeit |
| 913 | Druckmessstelle für Vor-Filter-Druck |
| 915 | Anschlussstelle für Heparin-Spritze |

| | |
|---|---|
| 917 | Filteranschluss |
| 919 | Citrat-Zuleitung |
| **1100** | Rückgabeleitung Calcium-Citrat |
| 1101 | Patientenkonnektor |
| 1103 | Schlauchklemme |
| 1105 | Calcium-Zuleitung |
| 1107 | venöser Blasenfänger |
| 1109 | Druckmessleitung |
| 1111 | Anschlussstück für Austauschflüssigkeit |
| 1113 | Probenentnahmestelle |
| 1115 | Filteranschluss |
| **1200** | Maschinenfront |
| 1201 | Druckmesseinheit |
| 1203 | Pumpe |
| 1205 | Halter für einen venösen Blasenfänger |
| 1207 | Luftblasendetektor |
| 1209 | Schlauchklemme |
| 1211 | Blutleckagesensor |
| 1213 | Halter für eine Calcium-Tropfkammer |
| 1215 | Calcium-Pumpe |
| 1217 | Halter für eine Citrat-Tropfkammer |
| 1219 | Citrat-Pumpe |

| | |
|---|---|
| ① | Konnektionsstelle Heparin |
| ② | Konnektionsstelle Calcium |
| ③ | Konnektionsstelle Citrat |
| p(art) oder PS1, p(PHF) oder PS2, p(ven) oder PS3 | Drucksensoren zum Erfassen des Blutdrucks |

| | |
|---|---|
| p(filt) oder PS4 | Drucksensor zum Erfassen eines Drucks des Dialysats |
| P1 | Blutpumpe |
| P5 | Pumpe für Calcium |
| P6 | Pumpe für Citrat |
| H. | Zugabestelle für Heparin |

| | |
|---|---|
| A1 | Signale von Behandlungsvorrichtung an Erfassungseinrichtung |
| A2 | |
| A3 | Signal von Erfassungseinrichtung an Behandlungsvorrichtung |
| E1 | Signal von Erfassungseinrichtung an Funktionseinrichtung |
| E2 | Signal von Funktionseinrichtung an |
| | Erfassungseinrichtung |
| B | Befestigung |
| P | Positionierung |

| | |
|---|---|
| S1 | Auswählen einer medizinischen Behandlungsoption an einer medizintechnischen Behandlungsvorrichtung |
| S2 | In-Kontakt-Bringen und/oder Verbinden einer medizintechnischen Funktionseinrichtung mit der Behandlungsvorrichtung |
| S3 | Erfassen des an der Funktionseinrichtung angeordneten Spezifikationsmerkmals mittels der Behandlungsvorrichtung |

| | |
|---|---|
| S4 | Erkennen und/oder Zuordnen einer mit dem Spezifikationsmerkmal verbundenen Funktion |
| S5 | Vergleichen der ausgewählten Behandlungsoption mit der anhand des Spezifikationsmerkmals erkannten Funktion |
| S6 | funktionelles Koppeln der Funktionseinrichtung und der Behandlungsvorrichtung, wenn die Behandlungsoption und die Funktion miteinander kompatibel sind |
| S7 | Starten definierter Abfragen |
| S8 | Ausgeben eines Alarms und/oder einer Anzeige, wenn die Behandlungsoption und die Funktion nicht miteinander kompatibel sind |

## Patentansprüche

1. Medizintechnische Funktionseinrichtung (300) mit wenigstens einem wenigstens eine medizintechnische Funktion oder Eignung der medizintechnischen Funktionseinrichtung (300) kennzeichnenden Spezifikationsmerkmal (400), wobei das Spezifikationsmerkmal (400) in, an oder auf der medizintechnischen Funktionseinrichtung (300) angebracht oder vorgesehen und ferner geeignet und/oder konfiguriert ist, um mittels einer mit der medizintechnischen Funktionseinrichtung (300) funktionell gekoppelten oder zu koppelnden medizintechnischen Behandlungsvorrichtung (100) oder einer mit letzterer in Signalübertragung verbundenen oder stehenden Einrichtung abgefragt zu werden.

2. Medizintechnische Funktionseinrichtung (300) nach Anspruch 1, ausgestaltet zu ihrer Verwendung zu einem extrakorporalen Blutbehandlungsverfahren.

3. Medizintechnische Funktionseinrichtung (300) nach einem der vorangegangenen Ansprüche, wobei das Spezifikationsmerkmal (400) an der Funktionseinrichtung (300) mittels Aufkleben, Aufdrucken, Beschichten, Einfärben, Durchfärben, Verschnappen oder Verrasten befestigt ist.

4. Medizintechnische Funktionseinrichtung (300) nach einem der vorangegangenen Ansprüche, mit wenigstens einem extrakorporalen Blutschlauchsatz, insbesondere einem in einem dazu ausgelegten und vorgesehenen Organizer (13) angeordneten, extrakorporalen Blutschlauchsatz, oder bestehend hieraus.

5. Medizintechnische Funktionseinrichtung (300) nach einem der Ansprüche 1 bis 3, wobei die Funktionseinrichtung (300) einen in einem Tray angeordneten, extrakorporalen Blutschlauchsatz aufweist oder hieraus besteht.

6. Medizintechnische Funktionseinrichtung (300) nach einem der vorangegangenen Ansprüche, ferner aufweisend wenigstens eine Citrat-Zugabestelle und/oder eine Calcium-Zugabestelle.

7. Medizintechnische Funktionseinrichtung (300) nach einem der vorangegangenen Ansprüche, ferner aufweisend wenigstens eine Heparin-Zugabestelle (35).

8. Medizintechnische Funktionseinrichtung (300) nach einem der vorangegangenen Ansprüche, wobei das Spezifikationsmerkmal (400) eine Farbmarkierung ist.

9. Medizintechnische Funktionseinrichtung (300) nach einem der vorangegangenen Ansprüche, wobei das Spezifikationsmerkmal (400) eine gelbe Farbmarkierung ist und die Funktionseinrichtung (300) eine Antikoagulationsleitung (231, 233) für Calcium und eine für Citrat aufweist.

10. Medizintechnische Funktionseinrichtung (300) nach einem der Ansprüche 1 bis 8, wobei das Spezifikationsmerkmal (400) eine blaue Farbmarkierung ist und die Funktionseinrichtung (300) eine Heparinleitung oder eine Zugabestelle (35) für Heparin aufweist.
